# EUROPEAN PATENT SPECIFICATION

(11) **EP 3 506 866 B1**
(45) Date of publication and mention of the grant of the patent: **27.07.2022**
(21) Application number: 17765326.8
(22) Date of filing: 29.08.2017
(51) Int. Cl.: A61F 13/00, A61M 1/00, A61M 27/00

(54) **WOUND CLOSURE DEVICES**
WUNDVERSCHLUSSVORRICHTUNGEN
DISPOSITIFS DE FERMETURE DE PLAIE

(30) Priority: 30.08.2016 US 201662381289 P; 12.09.2016 US 201662393477 P; 02.11.2016 US 201662416545 P; 23.06.2017 US 201762524090 P; 28.07.2017 US 201762538602 P
(43) Date of publication of application: 10.07.2019
(60) Divisional application: 22179746.7
(73) Proprietor: Smith & Nephew, Inc., Memphis, TN 38116 (US); University of Massachusetts, Boston, MA 02108 (US)
(72) Inventor: COLLINSON, Sarah Jenny, Hull HU3 2BN (GB); DUNN, Raymond M., Shrewsbury MA 01545 (US); HAMMOND, Victoria Jody, Hull HU3 2BN (GB); HARTWELL, Edward Yerbury, Hull HU3 2BN (GB); PHILLIPS, Marcus Damian, Hull HU3 2BN (GB); RICHARDSON, Mark, Hull HU3 2BN (GB); SAXBY, Carl Dean, Hull HU3 2BN (GB); STERN, Tim, Huddersfield West Yorkshire HD4 6SS (GB); SUGRUE, Michael, Hull HU3 2BN (GB); WILKINSON, Benjamin, Sheffield South Yorkshire S17 3LY (GB)
(74) Representative: Smith & Nephew
(86) International application number: PCT/US2017/049212
(87) International publication number: WO 2018/044949

(56) References cited:
- WO-A1-2014/014871
- WO-A1-2015/110409
- WO-A2-2015/061352

## Description

### BACKGROUND

### Field of the Invention

This application describes embodiments of systems for the treatment of wounds, specifically to aid in the closure of large wounds, in conjunction with the administration of negative pressure.

### Description of the Related Art

Negative pressure wound therapy has been used in the treatment of wounds, and in many cases can improve the rate of healing while also removing exudates and other deleterious substances from the wound site.

Abdominal compartment syndrome is caused by fluid accumulation in the peritoneal space due to edema and other such causes, and results in greatly increased intra-abdominal pressure that may cause organ failure eventually resulting in death. Causes may include sepsis or severe trauma. Treatment of abdominal compartment syndrome may require an abdominal incision to permit decompression of the abdominal space, and as such, a large wound may be created onto the patient. Closure of this wound, while minimizing the risk of secondary infections and other complications, and after the underlying edema has subsided, then becomes a priority. However, acute open abdominal conditions may be caused by other reasons in addition to compartment syndrome, as described further below.

Other large or incisional wounds, either as a result of surgery, trauma, or other conditions, may also require closure. For example, wounds resulting from sternotomies, fasciotomies, and other abdominal wounds may require closure. Wound dehiscence of existing wounds is another complication that may arise, possibly due to incomplete underlying fascial closure, or secondary factors such as infection.

Existing negative pressure treatment systems, while permitting eventual wound closure, still require lengthy closure times. Although these may be combined with other tissue securement means, such as sutures, there is also a risk that underlying muscular and fascial tissue is not appropriately reapproximated so as to permit complete wound closure. Further, when foam or other wound fillers are inserted into the wound, the application of negative pressure to the wound and the foam may cause atmospheric pressure to bear down onto the wound, compressing the foam downward and outward against the margins of the wound. This downward compression of the wound filler slows the healing process and slows or prevents the joining of wound margins. Additionally, inflammation of the fascia in the form of certain types of fasciitis can lead to rapid and excessive tissue loss, potentially meriting the need for more advanced negative pressure treatment systems. Accordingly, there is a need to provide for an improved apparatus, method, and system for the treatment and closure of wounds.

WO2015/110409 discloses the use of bespoke wound fillers in combination with negative pressure to treat a wound.

### SUMMARY

Embodiments of the present invention relate to negative pressure wound closure devices, that facilitate closure of a wound. It will be understood by one of skill in the art that the wounds described herein this specification may encompass any wound, and are not limited to a particular location or type of wound. The devices, operate to reduce the need for repetitive replacement of wound filler material currently employed and can advance the rate of healing. The devices, are simultaneously used with negative pressure to remove wound fluids.

The present invention is defined by the wound closure device of independent claim 1. Optional features of the invention are specified in the dependent claims. Any embodiments referred to and described herein which do not fall within the scope of the claims are not part of the present invention and should be considered as illustrative examples.

In some embodiments, the stabilizing structure may be at least partially curved along the horizontal plane. The top layer of the foam, the bottom layer of foam and/or the stabilizing structure may be curved along the length but not the width of the stabilizing structure, or curved along the width but not the length of the stabilizing structure. In some embodiments, the top layer of the foam, the bottom layer of foam and/or the stabilizing structure may be curved along both the length and the width of the stabilizing structure. The top layer of the foam, the bottom layer of foam, and/or the stabilizing structure may be dome-shaped. Each of the stabilizing structure, the top layer of foam and the bottom layer of foam may have an oval or oculiform shape. The stabilizing structure is configured to collapse more in a horizontal plane parallel to a length and a width of the stabilizing structure than in a vertical plane perpendicular to the horizontal plane. The length of the stabilizing structure may be greater than the width of the stabilizing structure. The wound closure device may further comprise a suction port configured to supply negative pressure to the wound. In some embodiments, a wound closure device may comprise a stabilizing structure for insertion into a wound, the stabilizing structure being at least partially curved along a horizontal plane parallel to a length and width of the stabilizing structure.

In some embodiments, the stabilizing structure may be curved only along the width but not the length of the stabilizing structure, or in some embodiments, the stabilizing structure may be curved along both the width and the length of the stabilizing structure. The stabilizing structure may comprise one or more detachable segments. The stabilizing structure may have an oval or oculiform shape. The stabilizing structure may be configured to collapse more in a horizontal plane parallel to a length and a width of the stabilizing structure than in a vertical plane perpendicular to the horizontal plane. The length of the stabilizing structure may be greater than the width of the stabilizing structure. The wound closure device may further comprise a suction port configured to supply negative pressure to the wound.

In some embodiments, a wound closure device may comprise a stabilizing structure for insertion into a wound, wherein the stabilizing structure is configured to be bent along a horizontal plane parallel to a length and a width of the stabilizing structure.

In some embodiments, the stabilizing structure may be configured to be bent reversibly. The stabilizing structure may be configured to be bent along the length of the stabilizing structure. The stabilizing structure may be configured to be bent in more than one direction, or substantially more in only one direction. The stabilizing structure may comprise a flexible material. The stabilizing structure may comprise one or more v-shaped cuts. The wound closure device may further comprise a top layer of foam for positioning above the stabilizing structure the top layer of foam conforming to the top of the stabilizing structure; and/or a bottom layer of foam for positioning below the stabilizing structure, the bottom layer of foam conforming to the bottom of the stabilizing structure. The stabilizing structure may be configured to collapse more in a horizontal plane parallel to a length and a width of the stabilizing structure than in a vertical plane perpendicular to the horizontal plane. The length of the stabilizing structure may be greater than the width of the stabilizing structure. The wound closure device may further comprise a suction port configured to supply negative pressure to the wound. In some embodiments, each of the stabilizing structure, the top layer of foam and the bottom layer of foam at least partially has an oval or elliptical shape.

In some embodiments, a wound closure device may comprise a stabilizing structure for insertion into a wound, the stabilizing structure comprising a top surface and a bottom surface defining a height defined as a thickness between the top and bottom surfaces, the stabilizing structure having a length and a width, wherein the stabilizing structure comprises a varying thickness across the length and/or width of the stabilizing structure.

In some embodiments, the stabilizing structure may comprise varying thickness across the length but not the width of the stabilizing structure, across the width but not the length of the stabilizing structure, or across both of the length and the width of the stabilizing structure. The thickness of the stabilizing structure may be thinner or thicker at the either or both ends along the length of the stabilizing structure. In some embodiments, the wound closure device may further comprise a top layer of foam for positioning above the stabilizing structure, the top layer of foam conforming to the top of the stabilizing structure; and/or a bottom layer of foam for positioning below the stabilizing structure, the bottom layer of foam conforming to the bottom of the stabilizing structure. The stabilizing structure may be configured to collapse more in a horizontal plane parallel to a length and a width of the stabilizing structure than in a vertical plane perpendicular to the horizontal plane. The length of the stabilizing structure may be greater than the width of the stabilizing structure. The wound closure device may further comprise a suction port configured to supply negative pressure to the wound. In some embodiments, each of the stabilizing structure, the top layer of foam and the bottom layer of foam at least partially has an oval or elliptical shape.

In some embodiments, a wound closure device may comprise:
a stabilizing structure for insertion into a wound,
wherein the stabilizing structure is configured to collapse more in a horizontal plane parallel to a length and a width of the stabilizing structure than in a vertical plane perpendicular to the horizontal plane; and
wherein the stabilizing structure comprises one or more detachable segments.

In some embodiments, the one or more detachable segments may comprise attachment elements. The stabilizing structure may comprise an inner segment at least partially surrounded by one or more detachable segments. The inner segment may comprise receiving elements configured to receive attachment elements of the one or more detachable segments. The one or more detachable segments may be configured to be removed only in a vertical direction or only in a horizontal direction. The one or more detachable segments may be configured to be remover in a vertical direction and/or a horizontal direction. The stabilizing structure may have an oculiform shape. The wound closure device may further comprise a suction port configured to supply negative pressure to the wound.

In some embodiments, a wound closure device may comprise:
a first stabilizing structure, the first stabilizing structure configured to collapse more in a horizontal plane parallel to a length and a width of the first stabilizing structure than in a vertical plane perpendicular to the horizontal plane; and
a second stabilizing structure positioned over the first stabilizing structure, the second stabilizing structure configured to collapse more in a horizontal plane parallel to a length and a width of the second stabilizing structure than in a vertical plane perpendicular to the horizontal plane.

In some embodiments, the second stabilizing structure may be attached to a top of the first stabilizing structure. The second stabilizing structure may comprise receiving elements configured to receive attachment elements of the first stabilizing structure. The first stabilizing structure may comprise receiving elements configured to receive attachment elements of the second stabilizing structure. The second stabilizing structure may be configured to be detachable from the first stabilizing structure. In some embodiments, the wound closure device comprises a third stabilizing structure positioned over the second stabilizing structure, the third stabilizing structure configured to collapse more in a horizontal plane parallel to a length and a width of the third stabilizing structure than in a vertical plane perpendicular to the horizontal plane. The third stabilizing structure may be attached to a top of the second stabilizing structure. The wound closure device may further comprise a suction port configured to supply negative pressure to the wound.

In some embodiments, a wound closure device may comprise:
a stabilizing structure for insertion into a wound;
a top layer of foam for positioning above the stabilizing structure; and
a suction port configured to be positioned above the top layer of foam, the suction port configured to supply negative pressure to the wound and configured to resist collapse under negative pressure.

In some embodiments, the suction port may be relatively rigid compared to the top layer of foam. The suction port may comprise a washer. The suction port may be pre-attached to the top layer of foam. The stabilizing structure may be configured to collapse more in a horizontal plane parallel to a length and a width of the stabilizing structure than in a vertical plane perpendicular to the horizontal plane.

In some embodiments, a wound closure device may comprise a stabilizing structure comprising a center line and two elliptiforms, the elliptiforms being mirror images of one another across the center line; wherein the stabilizing structure is configured to collapse more in a horizontal plane parallel to a length and a width of the stabilizing structure than in a vertical plane perpendicular to the horizontal plane.

In some embodiments, a wound closure kit may comprise:
one or more stabilizing structures, each stabilizing structure configured to collapse more in a horizontal plane parallel to a length and a width of the stabilizing structure than in a vertical plane perpendicular to the horizontal plane;
wherein each of the one or more stabilizing structures comprise a top foam layer and a bottom foam layer; and wherein each of the one or more stabilizing structures comprise attachment elements, the attachment elements configured to attach to receiving elements of a wound closure device.

In some embodiments, the wound closure device may be configured to collapse more in a horizontal plane parallel to a length and a width of the wound closure device than in a vertical plane perpendicular to the horizontal plane.

In some embodiments, a method of closing a sternum after a sternotomy may comprise: inserting a stabilizing structure within a gap in a sternum, the stabilizing structure configured to collapse more in a horizontal plane parallel to a length and a width of the stabilizing structure than in a vertical plane perpendicular to the horizontal plane; and positioning an organ protection layer under the stabilizing structure.

In some embodiments, the stabilizing structure may be configured to apply a closing force to the sternum. The method of closing a sternum may further comprise: covering the stabilizing structure with at least one drape sealed to skin surrounding the gap in the sternum; and applying negative pressure through the at least one drape to the wound via a source of negative pressure, wherein the application of negative pressure causes the clamping structure to collapse.

In some embodiments, and of the wound closure devices, described herein may further comprise a source of negative pressure. In some embodiments, the wound closure devices, described herein may further comprise one or more drapes configured to cover the stabilizing structure and form a seal around the wound. In certain embodiments, the stabilizing structure may comprise a plurality of cells provided side-by-side in a horizontal plane parallel to a length and width of the stabilizing structure, each cell defined by a plurality of walls extending in a vertical direction perpendicular to the horizontal plane.

Certain embodiments of stabilizing structures and related apparatuses and methods of treating a wound with reduced pressure, including pump and wound dressing components and apparatuses may be found in U.S. Provisional Application No. 62/393,477, filed September 12, 2016, and U.S. Provisional Application No. 62/416,545, filed November 2, 2016.

Other embodiments of wound closure devices, stabilizing structures and associated apparatuses are described below.

### BRIEF DESCRIPTION OF THE DRAWINGS

Other features and advantages of the present invention will be apparent from the following detailed description of the invention, taken in conjunction with the accompanying drawings of which:
Fig. 1 illustrates an embodiment of a negative pressure treatment system.
Figs. 2A-C illustrate multiple views of an embodiment of a stabilizing structure.
Figs. 3A-E illustrate multiple views of another embodiment of a stabilizing structure and a method of creating the stabilizing structure.
Fig. 4 illustrates an embodiment of an open abdominal wound.
Fig. 5 illustrates an embodiment of a step in a method of treating a wound.
Fig. 6 illustrates an embodiment of a step in a method of treating a wound.
Figs. 7A-C illustrate an embodiment of steps of a method of treating a wound.
Figs. 8A-B are photographs of steps of a method of treating a wound.
Figs. 9A-C depict an embodiment of steps of a method of treating a wound.
Fig. 10 contains photographs of embodiments of steps of a method of treating a wound.
Figs. 11A-G illustrate an embodiment of a method of treating a wound.
Fig. 12 illustrates an embodiment of a stabilizing structure.
Figs. 13A-C are drawings of an embodiment of a stabilizing structure.
Figs. 14A-D illustrate embodiments of stabilizing structures and foam layers.
Figs. 15A-E illustrate embodiments of stabilizing structure with outer shell(s) or detachable segment(s).
Figs. 16A-D illustrate embodiments of stabilizing structures with detachable segments.
Fig. 17 illustrates an embodiment of a stabilizing structure comprising extending cells and recesses.
Figs. 18A-E illustrate embodiments of stabilizing structures with detachable segments.
Fig. 19 illustrates an embodiment of a wound closure device with stackable stabilizing structures.
Figs. 20A-C illustrate an embodiment of a method of closing a sternal opening with a wound closure device or stabilizing structure.
Figs. 21A-D illustrate an embodiment of a stabilizing structure curved along only its width.
Figs. 22A-D illustrate an embodiment of a stabilizing structure curved along both its width and its length.

### DETAILED DESCRIPTION

Embodiments disclosed in this section or elsewhere in this specification relate to apparatuses and methods of treating a wound with reduced pressure, including pump and wound dressing components and apparatuses. The apparatuses and components comprising the wound overlay and packing materials, if any, are sometimes collectively referred to in this section or elsewhere in this specification as dressings.

It will be appreciated that throughout this specification reference is made to a wound. It is to be understood that the term wound is to be broadly construed and encompasses open and closed wounds in which skin is torn, cut or punctured or where trauma causes a contusion, or any other superficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, abdominal wounds or other large or incisional wounds, either as a result of surgery, trauma, sternotomies, fasciotomies, or other conditions, dehisced wounds, acute wounds, chronic wounds, subacute and dehisced wounds, traumatic wounds, flaps and skin grafts, lacerations, abrasions, contusions, burns, electrical burns, diabetic ulcers, pressure ulcers, stoma, surgical wounds, trauma and venous ulcers or the like.

As is used in this section or elsewhere in this specification, reduced or negative pressure levels, such as -X mmHg, represent pressure levels that are below standard atmospheric pressure, which corresponds to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than -X mmHg corresponds to pressure that is closer to atmospheric pressure (e.g., ---40 mmHg is less than - 60 mmHg). Negative pressure that is "more" or "greater" than -X mmHg corresponds to pressure that is further from atmospheric pressure (e.g., -80 mmHg is more than -60 mmHg).

The negative pressure range for some embodiments of the present disclosure can be approximately -80 mmHg, or between about -10 mmHg and -200 mmHg. Note that these pressures are relative to normal ambient atmospheric pressure. Thus, -200 mmHg would be about 560 mmHg in practical terms. In some embodiments, the pressure range can be between about -40 mmHg and -150 mmHg. Alternatively, a pressure range of up to -75 mmHg, up to -80 mmHg or over -80 mmHg can be used. Also in other embodiments a pressure range of below -75 mmHg can be used. Alternatively, a pressure range of over approximately -100 mmHg, or even -150 mmHg, can be supplied by the negative pressure apparatus. In some embodiments, the negative pressure range can be as small as about -20 mmHg or about -25 mmHg, which may be useful to reduce fistulas. In some embodiments of wound closure devices described here, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some embodiments, negative pressure may be varied over time for example using a sinusoidal wave, square wave, and/or in synchronization with one or more patient physiological indices (e.g., heartbeat).

Examples of such applications where additional disclosure relating to the preceding descriptions may be found include U.S. Patent No. 8,235,955, titled "Wound treatment apparatus and method," issued August 7, 2012 and U.S. Patent No. 7,753,894, titled "Wound cleansing apparatus with stress," issued July 13, 2010.

Other applications that may contain teachings relevant for use with the embodiments described in this section or elsewhere in this specification may include Application Serial No. 12/886,088, titled "Systems And Methods For Using Negative Pressure Wound Therapy To Manage Open Abdominal Wounds," filed September 20, 2010, published as US 2011/0213287; Application Serial No. 13/092,042, titled "Wound Dressing And Method Of Use," filed April 21, 2011, published as US 2011/0282309; and Application Serial No. 13/365,615, titled "Negative Pressure Wound Closure Device," filed February 3, 2012, published as US 2012/0209227.

Still more applications that may contain teachings relevant for use with the embodiments described in this specification are Application Serial No. 13/942,493, titled "Negative Pressure Wound Closure Device," filed July 15, 2013, published as US 2014/0180225; PCT App. No. PCT/US2013/050619, filed July 16, 2013 titled "Negative Pressure Wound Closure Device," published as WO 2014/014871 A1; PCT App. No. PCT/US2013/050698, filed July 16, 2013 titled "Negative Pressure Wound Closure Device," published as WO 2014/014922 A1; PCT App. No. PCT/IB2013/01555, titled "Devices and Methods for Treating and Closing Wounds with Negative Pressure," filed May 5, 2013, published as WO 2013/175309 A1; PCT App. No. PCT/US2014/025059, titled "Negative Pressure Wound Closure Device and Systems and Methods of Use in Treating Wounds with Negative Pressure," filed March 12, 2014, published as WO 2014/165275 A1; and PCT App. No. PCT/GB2014/050746, "Compressible Wound Fillers and Systems and Methods of Use In Treating Wounds With Negative Pressure," filed Mar 13, 2014, published as WO 2014/140578 A1, and "Negative Pressure Wound Closure Device," filed Oct 21, 2014, and published as PCT/US2014/061627.

It will be understood that throughout this specification, in some embodiments, reference is made to an elongate, elongated or longitudinal strip or strips. It is to be understood that these terms are to be broadly construed and refer in some embodiments to an elongate material having two parallel or substantially parallel faces, where in cross-section a thickness of the material as measured perpendicular to the faces is relatively smaller than a height of the material measured parallel to the faces. While in some embodiments the strips may be constructed from discrete lengths of material, in other embodiments the strips may simply refer to elongate portions of an overall structure having two parallel or substantially parallel faces. The strips in some embodiments have a rectangular or generally rectangular-shaped faces, wherein a length of the face is longer than the height of the face. In some embodiments, the length of the face may be more than 2 times, 4 times, 6 times, 8 time, 10 times, 12 times or more greater than the height of the face.

As used in this section or elsewhere in this specification, the term "horizontal," when referring to a wound, indicates a direction or plane generally parallel to the skin surrounding the wound. The term "vertical," when referring to a wound, generally refers to a direction extending perpendicular to the horizontal plane. The term "longitudinal," when referring to a wound, generally refers to a direction in the horizontal plane taken in a direction along which the wound is longest. The term "lateral," when referring to a wound, generally refers to a direction in the horizontal plane perpendicular to the longitudinal direction. The terms "horizontal," "vertical," "longitudinal" and "lateral" may also be used to describe the stabilizing structures and wound closure devices described throughout this specification. When describing these structures or devices, these terms should not be construed to require that the structures or devices necessarily be placed into a wound in a certain orientation, though in certain embodiments, it may be preferable to do so.

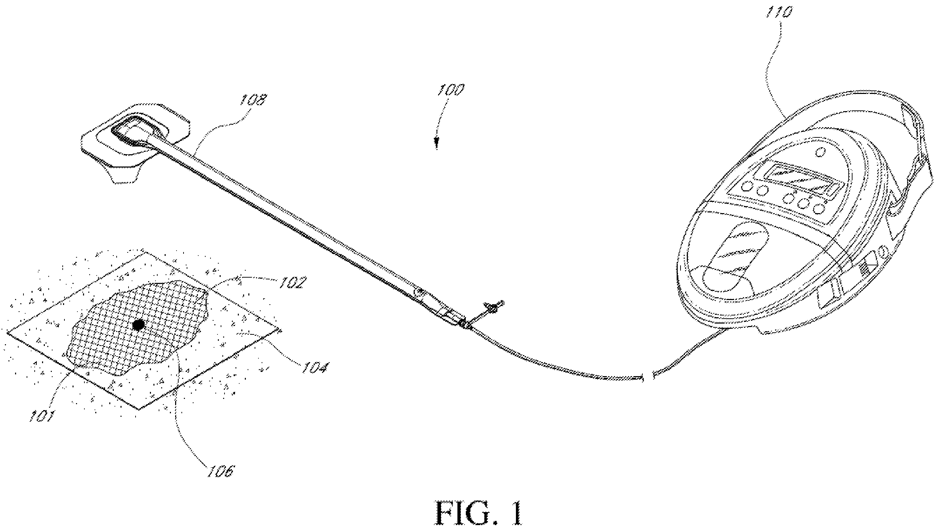

Figure 1 illustrates an embodiment of a negative pressure treatment system 100 that comprises a wound packer 102 inserted into a wound 101. The wound packer 102 may comprise porous materials such as foam, and in some embodiments may comprise one or more embodiments of wound closure devices described in further detail in this section or elsewhere in this specification. In some embodiments, the perimeter or top of any wound closure device inserted into the wound 101 may also be covered with foam or other porous materials. A single drape 104 or multiple drapes may be placed over the wound 101, and is preferably adhered or sealed to the skin on the periphery of the wound 101 so as to create a fluid-tight seal. An aperture 106 may be made through the drape 104 which can be manually made or preformed into the drape 104 so as to provide a fluidic connection from the wound 101 to a source of negative pressure such as a pump 110. Preferably, the fluidic connection between the aperture 106 and the pump 110 is made via a conduit 108. In some embodiments, the conduit 108 may comprise a RENASYS^{®} Soft Port^{™}, manufactured by Smith & Nephew. Of course, in some embodiments, the drape 104 may not necessarily comprise an aperture 106, and the fluidic connection to the pump 110 may be made by placing the conduit 108 below the drape. In some wounds, particularly larger wounds, multiple conduits 108 may be used, fluidically connected via one or more apertures 106.

In some embodiments, the drape 104 may be provided with one or more corrugations or folds. Preferably, the corrugations are aligned along the longitudinal axis of the wound, and as such may support closure of the wound by preferentially collapsing in a direction perpendicular to the longitudinal axis of the wound. Such corrugations may aid in the application of contractile forces parallel to the wound surface and in the direction of wound closure. Examples of such drapes may be found in Application Serial No. 12/922,118, titled "Vacuum Closure Device," filed November 17, 2010 (published as US 2011/0054365).

In use, the wound 101 is prepared and cleaned. In some cases, such as abdominal wounds, a non- or minimally-adherent organ protection layer (not illustrated) may be applied over any exposed viscera. The wound packer 102 is then inserted into the wound, and is covered with the drape 104 so as to form a fluid-tight seal. A first end of the conduit 108 is then placed in fluidic communication with the wound, for example via the aperture 106. The second end of the conduit 108 is connected to the pump 110. The pump 110 may then be activated so as to supply negative pressure to the wound 101 and evacuate wound exudate from the wound 101. As will be described in additional detail below and in relation to the embodiments of the foregoing wound closure devices, negative pressure may also aid in promoting closure of the wound 101, for example by approximating opposing wound margins.

Any structure or component disclosed herein this section or elsewhere in the specification may comprise a radiopaque material. A radiopaque material advantageously allows a clinician to more easily find pieces of the wound closure device that may have come loose from the structure and become lost in the wound. Some examples of radiopaque materials include barium sulfate, bismuth trioxide, bismuth subcarbonate, bismuth oxychloride, and tungsten.

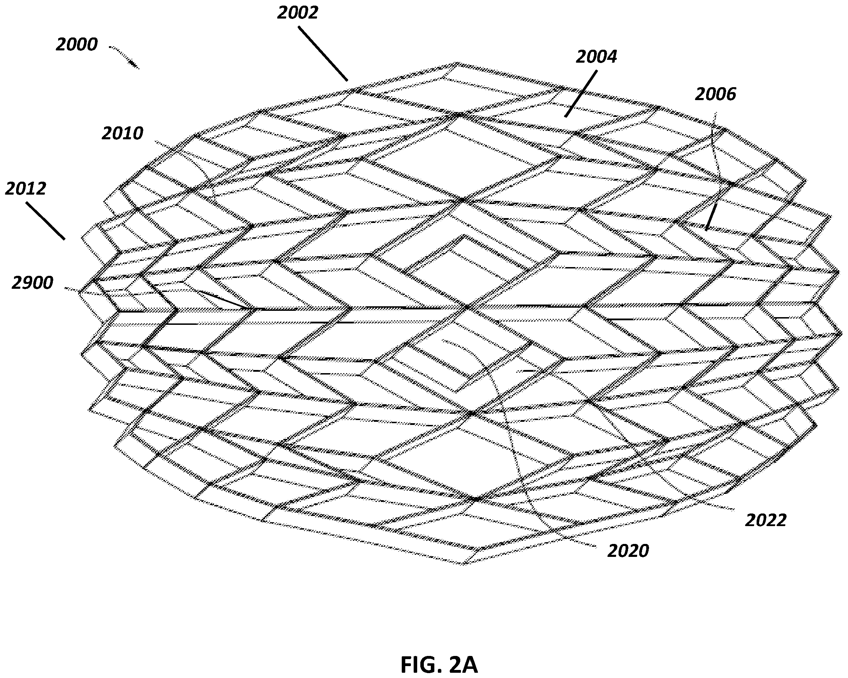

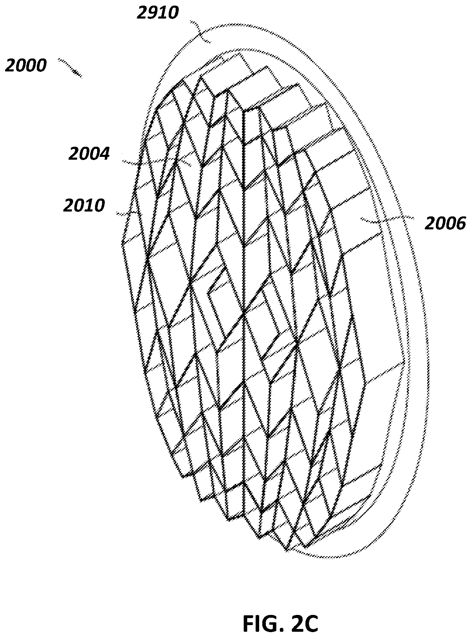

### Stabilizing Structures and Wound Closure Devices - of Figure 2A-3E

Figure 2A is a drawing of an embodiment of a stabilizing structure 2000 comprising a plurality of elongate strips 2006 arranged in parallel or semi-parallel, whose longitudinal length can be aligned with the longitudinal axis of a wound. In embodiments, the elongate strips 2006 may also be arranged in a non-parallel fashion. The various cells within this stabilizing structure 2000 may have a variety of shapes and sizes. As will be described in greater detail below, the length and shape of the elongate strips 2006, intervening members 2010, and cells 2004 may be designed so as to facilitate greater closure of the stabilizing structure. In certain embodiments, the junctions 2900 between the elongate strips and intervening members may be thinned to better facilitate rotation and closure of the stabilizing structures. In some embodiments, the stabilizing structure is tearable, such that the structure may be shaped into the shape of a wound. As described elsewhere in the specification, tears may be completed at the intersections between intervening members and elongate strips or at any suitable location along the elongate strip or intervening member.

All stabilizing structures described herein this section or elsewhere in the specification may be fashioned to accommodate any size of wound. However, to better accommodate the needs of the clinical environment, in certain embodiments, the stabilizing structures described herein may be provided in a pack of two sizes, one smaller stabilizing structure and one larger stabilizing structure about 1.25 times as larger, about 1.5 times as large, about 1.75 times as large, about 2 times as larger, about 2.5 times as larger, about 3 times as large, about 4 times as large, about 5 times as large, or more than about 5 times as large. In some embodiments, the pack may comprise more than two sizes, such as three sizes, four sizes, five sizes, or more than five sizes. The stabilizing structures within the pack may be of a variety of sizes in relation to one another such as the ratios described above.

In certain embodiments, the stabilizing structure 2000 can collapse in any manner described in this section or elsewhere in this specification with or without the application of negative pressure. For example, the stabilizing structure may collapse significantly more in one plane than in another plane upon application of negative pressure. In some embodiments, the stabilizing structure is configured to collapse more in a horizontal plane parallel to the length and width of the stabilizing structure than in a vertical plane perpendicular to the horizontal plane. In embodiments, particular rows may collapse in a first direction, while another row may collapse in the same or an opposing direction. In certain embodiments, the stabilizing structure may collapse along the width of the stabilizing structure while remaining relatively rigid along the length of the stabilizing structure and in the vertical direction.

The stabilizing structure may be comprised of any materials described in this section or elsewhere in this specification, including: flexible plastics such as silicone, polyurethane, rigid plastics such as polyvinyl chloride, semi-rigid plastics, semi-flexible plastics, biocompatible materials, composite materials, metals, and foam. In certain embodiments, the stabilizing structure may comprise a radio opaque material, to more readily allow a clinician to find pieces of the stabilizing structure within the wound.

Returning to Figure 2A, stabilizing structure 2000 may have an outer perimeter that defines an at least partially elliptical shape. As described above, stabilizing structure 2000 may comprise a plurality of cells 2004 provided side-by-side, each cell defined by one or more walls, each cell having a top end and a bottom end with an opening extending through the top and bottom ends. As with the other stabilizing structures described herein this section and elsewhere in the specification, the stabilizing structure 2000 is configured to collapse by collapsing one or more cells 2004. In some embodiments, the cells are all of the same approximate shape and size; however, in other embodiments, the cells are of different shapes and sizes. In some embodiments, the stabilizing structures as described herein this section or elsewhere in the specification may be domed, such that the central portion of the stabilizing structure bulges upward. For example, a lower portion of the stabilizing structure may be concave, while an upper portion of the stabilizing structure is convex.

The elongate strips 2006 may be made from one single material, such as those described elsewhere in the specification, or the elongate strips may be made from multiple materials. For example, elongate strips 2006 may comprise sections of more rigid material and sections of more flexible material. The elongate strips 2006 may be curved along their length so as to facilitate the curved outer perimeter of the stabilizing structure 2000. The elongate strips may be curved along their lengths outward away from a center of the stabilizing structure 2000. The arch of the curves of the elongate strips 2006 may vary considerably, with some strips 2006 being highly curved while other are minimally curved or even straight.

Similarly, the stabilizing structure 2000 can further comprise a plurality of intervening members 2010 connected to the elongate strips 2006. The intervening members 2010 may all be of a similar shape and size or they may be of a variety of shapes and sizes. The intervening members may be constructed from any material disclosed herein this section or elsewhere in the specification. Further, the intervening members may be constructed from multiple materials.

Advantageously, the elliptical shape of stabilizing structure 2000 may allow the structure to better accommodate the shape of the wound. Most wounds are in shapes that are rounded, thus, an elliptically shaped stabilizing structure 2000 may better fit into a wound.

In embodiments, the outer perimeter 2002 may have a reduced edge 2012 so as to facilitate collapse of the stabilizing structure. By removing mass of the stabilizing structure at reduced edge 2012, the stabilizing structure can collapse more freely at reduced edge 2012, thus allowing for a better fit within the wound. Further, by reduced the mass at reduced edge 2012, there may be less pinching of the surrounding tissue during and after collapse of the stabilizing structure 2000.

The stabilizing structure 2000 and all stabilizing structures and wound closure devices described in this section or elsewhere in this specification can collapse on a variety of timescales in a dynamic fashion. In certain embodiments, the majority of the collapse may occur within the first few minutes upon application of negative pressure. However, after the initial collapse, the stabilizing structure or wound closure device may continue to collapse at a much slower rate, thereby applying increasing longitudinal tension over a long period of time and drawing the edges of the wound closer together. By slowly drawing the wound edges closer together over time, the stabilizing structure or wound closure device allows the surrounding healing tissue to remodel synergistically with the closure of the device or stabilizing structure. Slow, dynamic wound closure may allow the surrounding tissue to heal at an accelerated rate, because the collapsing structure or device slowly brings the edges of the wound closer together without stressing the newly formed or weakened tissue too quickly.

In some embodiments, the stabilizing structures described in this section or elsewhere in this specification can be placed into a wound for a period of time and then removed or replaced with another stabilizing structure. For example, a stabilizing structure could be inserted into a wound for a period of time, promoting closure of the wound by drawing the edges closer together. After a period of time has passed, the stabilizing structure can be replaced by a stabilizing structure of a different size or collapsibility, for example a stabilizing structure of a smaller size or decreased density. This process could be repeated over and over, thereby continuously drawing the edges of the wound together over time and allowing for continuing repair and remodeling of the surrounding tissue. In certain embodiments, the stabilizing structure is configured to remain in the wound for at least about less than 1 hour, at least about 1 hour, at least about 2 hours, at least about 4 hours, at least about 6 hours, at least about 8 hours, at least about 12 hours, at least about 24 hours, at least about 2 days, at least about 4 days, at least about 6 days, at least about 1 week, at least about 2 weeks, at least about 3 weeks, or more than 3 weeks.

In certain embodiments, up to 90% of the collapse of the stabilizing structure or wound closure device may occur within the first few minutes upon application of negative pressure, while the remaining 10% of the collapse may occur slowly over a period of many minutes, hours, days, weeks, or months. In other embodiments, up to about 80% of the collapse, up to about 70%, up to about 60%, up to about 50%, up to about 40%, up to about 30%, up to about 20%, up to about 10%, or about 0% of the collapse will occur immediately within the first few minutes upon application of negative pressure while the remainder of the collapse occurs at a much slower rate such as over the course of many minutes, hours, days weeks, or months. In other embodiments, the stabilizing structure can collapse at a variable rate. In some embodiments, the entirety of the collapse occurs at a slowed rate, while in other embodiments the entirety of the collapse occurs almost immediately within the first few minutes. In further embodiments, the collapse can occur at any rate and the rate can vary over time. In certain embodiments, the rate of collapse can be altered in a variable fashion by adding and/or removing portions of the structure or by controlling the application of negative pressure and irrigant fluid.

Returning to Figure 2A, in some embodiments, the pattern of the stabilizing structure 2000 is designed in such a way as to facilitate maximum closure of the stabilizing structure. Preferably, maximum closure is in a direction perpendicular to the length of the elongate members and within the horizontal plane. As will be described in greater detail below, greater closure may be achieved by varying the length of the elongate strips 2006, the length of the intervening members 2010, and the shape of the cells 2004. The shape of the cells 2004 may comprise any shape described herein this section or elsewhere in the specification. For example, as depicted in Figure 2A, the cells 2004 may be diamond-shaped or parallelepiped with smaller diamond-like shapes 2020 located within larger diamonds 2022. Such a construction may provide greater overall closure of the stabilizing device 2000 to provide for maximum closure of the wound. Additionally, the smaller diamond-like shapes 2020 located within larger diamonds 2022 can spread the load over a greater area reducing the chance of damage to the tissue structures below the matrix. This construction can also reduce the likelihood of the foam or the drape being pulled into the matrix and preventing closure of the wound.

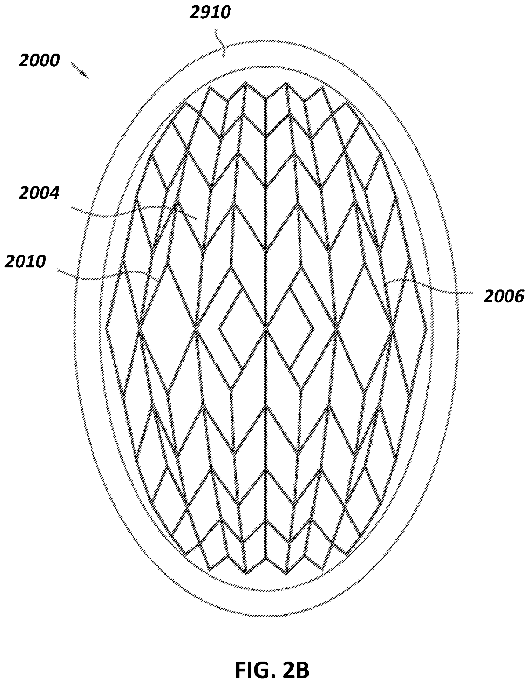

Figures 2B-C are illustrations of different views of the stabilizing structure embodiment of Figure 2A. As described above in relation to Figure 2A, the stabilizing structure comprises cells 2004, intervening members 2010, and elongate strips 2006; however, here a simulated shape of a wound 2910 is also included for comparison.

Any of the stabilizing structures described herein this section or elsewhere in the specification may be constructed from any suitable means. For example, the stabilizing structures may be constructed via molding or may be printed directly using 3D printing technology. In certain embodiments, the stabilizing structures of Figures 2A-C may be constructed from a single polymer via 3D printing. In some embodiments, the stabilizing structures may be constructed from one polymer, two polymers, three polymers, or more than three polymers. The stabilizing structures may be constructed from any material disclosed herein this section or elsewhere in the specification. The stabilizing structure can be made by cutting the structure out of a solid block of material. Methods used for cutting can include, for example, water jet cutting, laser cutting, or die cutting. The stabilizing structures may be cut to size along the walls of the cells 2004. For example, the intervening members along the outside face of elongate strips 2006 can be cut off to appropriately size the stabilizing structure. The stabilizing structure may be cut along the walls, along any portions of the elongate strips, and/or along any portions of the intervening members.

In some embodiments, the stabilizing structure 2000 of Figures 2A-C can be configured to include perforations or detachable sections that allow portions of the device to separate from the remainder of the device. For example, perforations may be incorporated into the joints 2900 between various cells 2004 contained within the stabilizing structure 2000, allowing for the removal of individual rows or cells to alter the shape of the stabilizing structure 2000.

Applicable to all stabilizing structures or wound closure devices described in this section or elsewhere in the specification, the stabilizing structure or wound closure device may be tearable such that the stabilizing structure may be shaped into the shape of a wound. In some embodiments, the stabilizing structure may be torn at the intersections between intervening members and elongate strips, while in further embodiments, the elongate strips or intervening members may be torn at any suitable position.

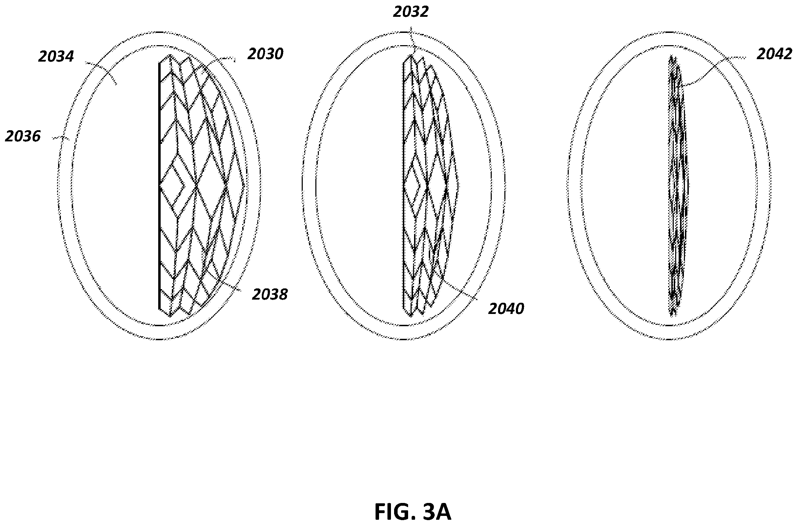

Figures 3A-E depict methodologies for generating the design of a stabilizing structure, such as the stabilizing structures of Figures 2A-C. To facilitate various types of closure (for example, maximum closure) the shape, size, and location of the elongate strips, intervening members, and cells may be determined via various methods. For example, as depicted in Figure 3A, each collapsible cell 2030 has four sides, and each intersection between an intervening member(s) and/or elongated strip(s) may be modeled via pin-joints 2032. Further, the entirety of stabilizing structure 2034 may be modeled inside of an oval wound model 2036. As depicted in Figure 3A, the stabilizing structure 2034 may be modeled to collapse from an open state 2038 to a semi-collapsed state 2040, to a fully collapsed state 2042. In some clinical scenarios, maximum closure down to a completely flattened stabilizing structure may be desirable to maximize wound closure by drawing the edges of the wound as close together as possible.

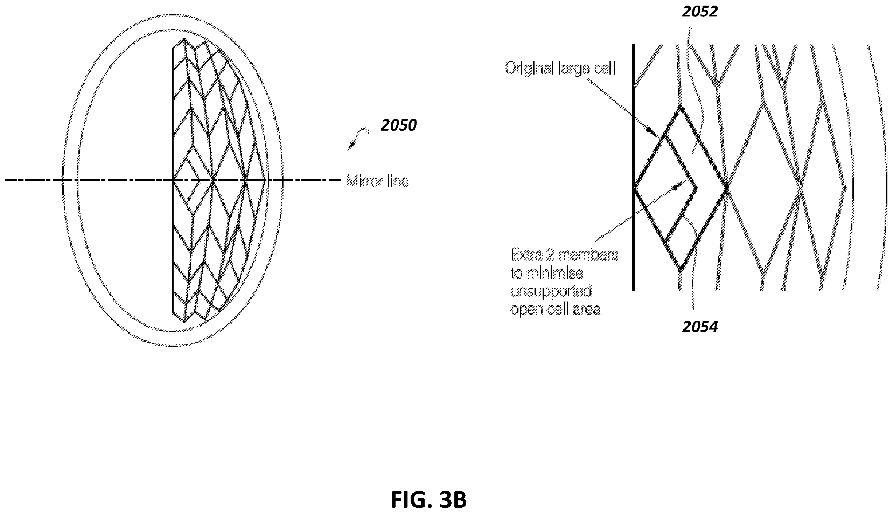

As illustrated in Figure 3B, in certain embodiments, the process of determining the optimal shape, size, and location of the elongate strips, intervening members, and cells for wound closure may be facilitated by modeling the stabilizing structure as a mirrored pattern on opposite sides of a mirror line 2050 (which may also be referred to as the transverse axis, perpendicular to a longitudinal axis of the stabilizing structure), thereby making the curve and collapse of the stabilizing structure symmetrical. The mirror axis may be along the minor axis or it may be along the major axis of the stabilizing structure. Alternatively, the mirror line may be located in any suitable location within the stabilizing structure, such as diagonally across the stabilizing structure. In certain embodiments, this method may lead to large diamond-shaped cells near the center line. These large diamond-shaped structures 2052 may be further subdivided to further support the stabilizing structure by including smaller diamond shapes 2054 within larger shapes. In some embodiments, these smaller shapes 2054 within a larger shape 2052 may comprise any shape disclosed herein this section or elsewhere in the specification. The larger cells may be further subdivided by two smaller shapes, three smaller shapes, four smaller shapes, or more than four smaller shapes. It will be understood by one of skill in the art that the mirror line need not be confined to a line perpendicular to the longitudinal orientation of the wound. Instead, the mirror line may be located along the longitudinal axis of the wound or at an angle to the longitudinal axis of the wound. In some embodiments, the stabilizing structure may contain multiple mirror lines, thereby having multiple subsections that are symmetrical or different.

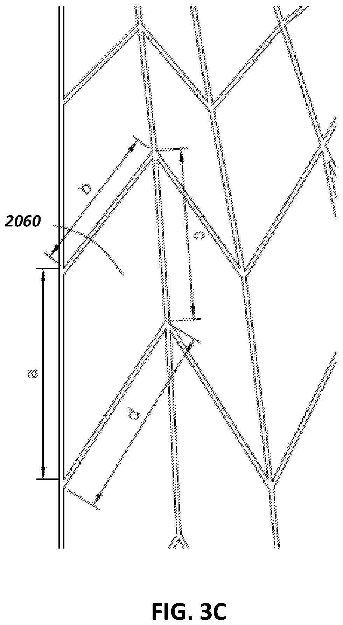

As illustrated in Figure 3C, for a four-sided cell to collapse, it must follow a simple formula: a + b = c + d, where a, b, c, and d are the lengths of individual sides of a single cell within the stabilizing structure such as the cell 2060 of Figure 3C. When members c and b collapse together, then d and a collapse together. Such a formula may be the basis for developing a pattern for a stabilizing structure that maximizes collapsibility.

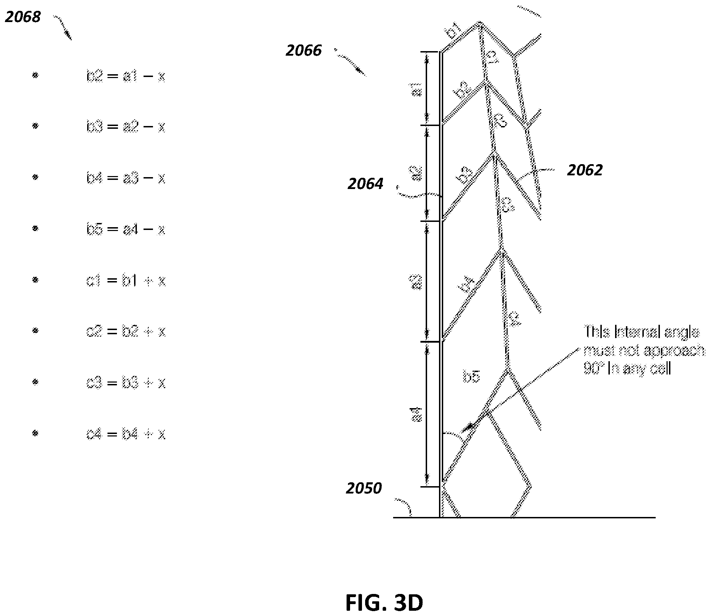

Figure 3D illustrates an expansion of the concept described in Figure 3C. By using the base formula a + b = c + d, the elongate strips were progressively lengthened (a4 > a3 > a2 > al) towards the horizontal mirror line 2050, thereby achieving a curve in the stabilizing structure while preventing any of the intervening members 2062 from becoming perpendicular to the elongate strips 2064 (i.e. having an internal angle of 90 degrees). As illustrated in Figure 3D, a value for bl may be chosen, at which point an arbitrary offset value x may also be chosen to ease the construction of the various cell geometries. Using the progressive values for a1 through a4, illustrated visually in Figure 3D 2066, values for b1-b4 may be calculated 2068. Using calculated values derived from equations 2068 for the various walls of the individual cells allows for the design of a stabilizing structure that collapses completely, such as those depicted in Figures 3A-B.

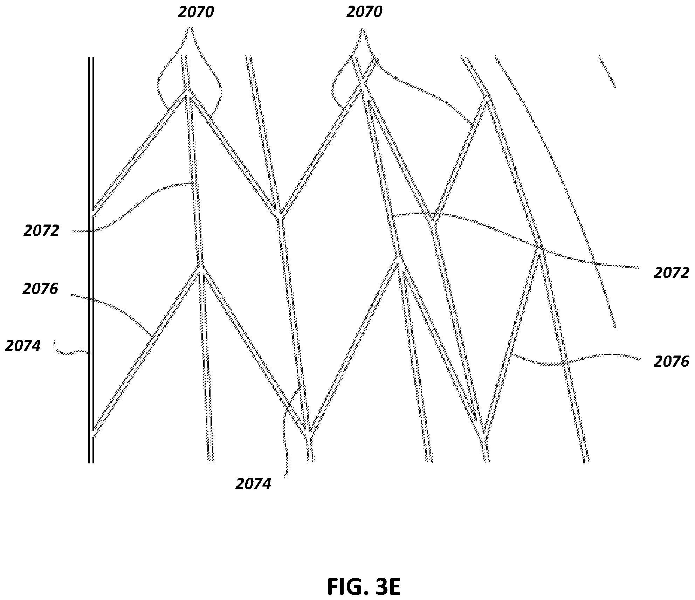

In some embodiments, a method for generating a stabilizing structure design may include steps to speed up the initial geometry construction. For example if all members from left to right in a specific row, as visualized by intervening members 2036 in Figure 3E, a pattern then emerges where alternating vertical members are also the same length. Walls of the same length are indicated by their respective labels 2070, 2072, 2074, and 2076. Once the initial design is generated then individual cells may be modified by lengthening, shortening, removing or inserted according to the formulas of Figure 3D to achieve the desired shape of the overall stabilizing structure. The method of use of the wound closure device is not part of the present invention

### Wound Closure and Treatment Methods of F mures 4-11G

The stabilizing structures and/or wound closure devices described in this section or elsewhere in this specification may be used in conjunction with methods or systems for the closure of a wound. In some embodiments of methods of use for closure of a wound, one or more of the stabilizing structures or wound closure devices of any of the embodiments described in this section or elsewhere in this specification is placed into a wound. In some embodiments, an organ protection layer may be provided in the wound before placement of the stabilizing structure. In certain embodiments, foam or other porous material may be placed in the wound along with the stabilizing structure or wound closure device, either below, above, or surrounding the stabilizing structure or wound closure device. Foam or other porous material may also surround the perimeter of the stabilizing structure or wound closure device. The stabilizing structure or wound closure device may be configured to collapse in any manner as described in this section or elsewhere in this specification, for example by having a particular size and shape, or by comprising a certain volume of foam or other porous material within the cells of the structure. The stabilizing structure or wound closure device may further be altered in any manner described in this section or elsewhere in this specification so as to better accommodate the shape of the wound. After placement in the wound, the stabilizing structure or wound closure device can be sealed by a fluid-tight drape. The fluid-tight drape can comprise a port configured for the application of negative pressure. A source of negative pressure may then be connected to the port and negative pressure may be applied to the wound. The stabilizing structure or wound closure device may be replaced over time by stabilizing structures or wound closure devices of various shapes and sizes as desired to best promote wound healing.

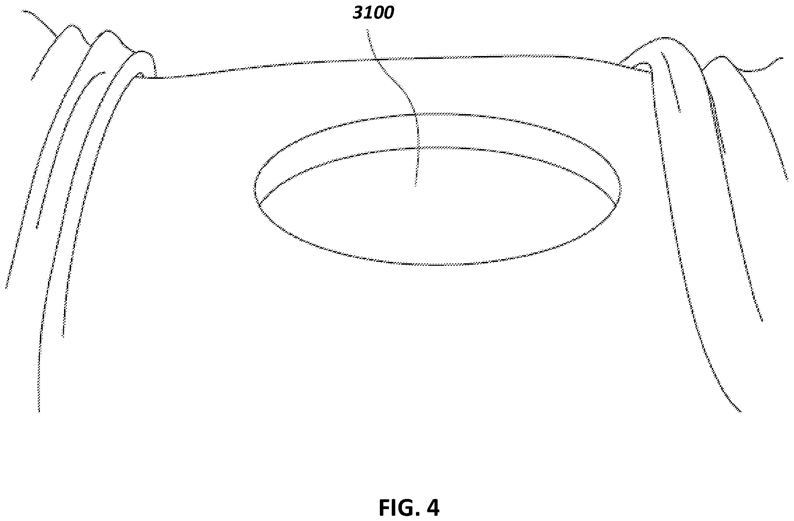

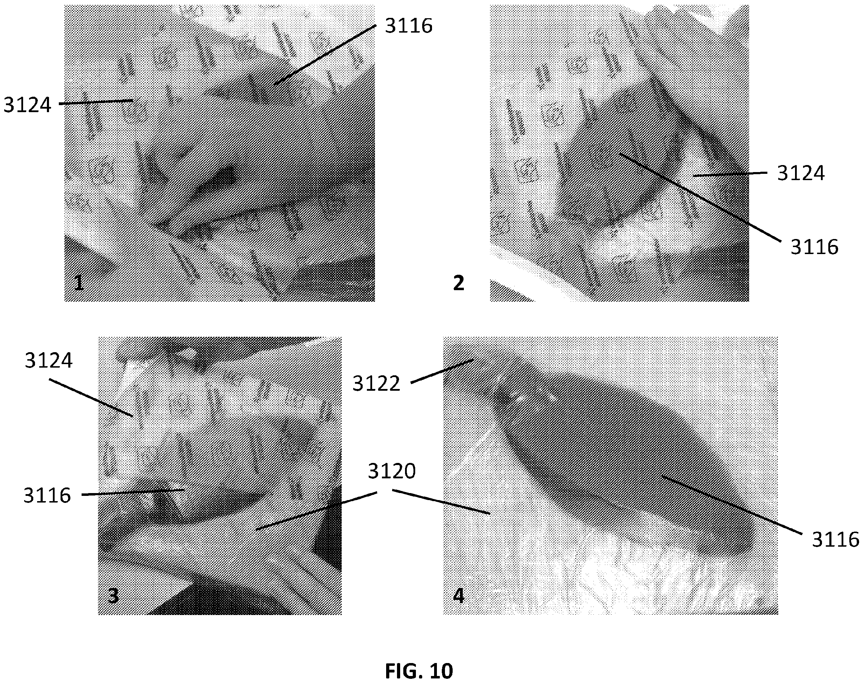

Figures 4-11G are photographs and illustrations depicting embodiments of methods for the treatment of a wound that utilize a wound closure device comprising a stabilizing structure as described herein this section and elsewhere in the specification. To better illustrate non-limiting embodiments of the methods, numbers have been added to the steps of Figure 10 to allow the reader to more easily follow these steps of the method. However, the steps can be performed in any order, and any numbering system is for clarity only. Further, in some embodiments, different steps of these methods may be excluded. In other embodiments, additional steps may be added to the methods based on methods described herein this section and elsewhere in the specification. The porous layers and structures described in this section may be of any material or structure described elsewhere in the specification, such as foam.

Figure 4 depicts an embodiment of an open wound 3100 prior to treatment with a wound closure device as will be described in much greater detail below. The open wound of Figure 4 is similar to the wounds described elsewhere in the specification, particularly as relate to Figure 1. In some instances, as described elsewhere in the specification, such a wound may be produced via a surgical incision or other means.

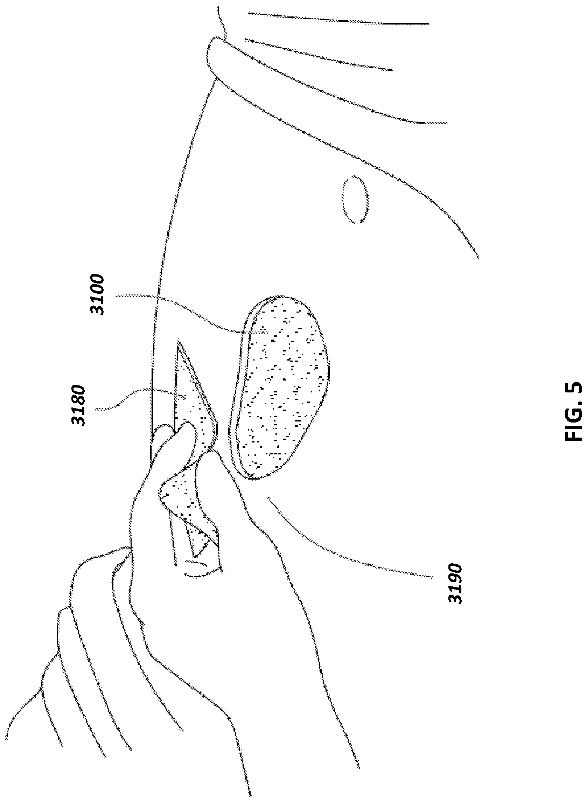

Figure 5 depicts an embodiment of an initial step in a method for the treatment of an open wound 3100 with a wound closure device. Before treatment, the wound may be cleaned with a pad 3180 and the skin 3190 prepared for application of a wound closure device, such as those described in relation to Figures 2A-3E.

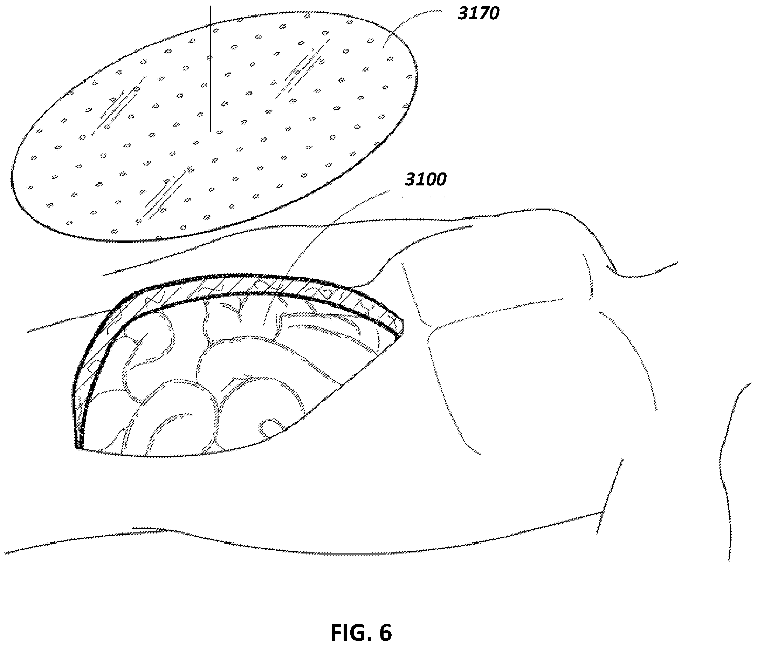

Figure 6 depicts an embodiment of an early step in a method for the treatment of an open wound 3100. In some embodiments, a tissue protection layer 3170 may be placed over the wound to protect the underlying tissues from the rigors of negative pressure wound therapy or other potential harms. Accordingly, certain embodiments provide for a tissue protection layer 3170 which may be cut to size to be placed over the wound site 3100. The tissue protection layer 3170 can be a material which will not adhere to the wound site or to the exposed viscera in close proximity. Such a tissue protection layer may be constructed from any suitable material such as a biocompatible polymer. For example, organ protection layers manufactured by Smith & Nephew and sold under the brand RENASYS^{®} may act as tissue protection layers and be placed over the abdominal cavity and/or wound bed 3100 and tucked over the peritoneal gutter. In further examples, materials such as the fluoropolymer polytetrafluoroethylene (PTFE) may be applicable as these materials are generally non-adherent and used in surgical grafts. In one embodiment, the tissue protection layer is permeable. For example, the tissue protection layer 3170 can be provided with openings, such as holes, slits, or channels, to allow the removal of fluids from the wound site 3100 or the transmittal of negative pressure to the wound site 3100. In further embodiments, the tissue protection layer may be used over non-abdominal wounds on other areas of the body, such as the leg, arm, shoulder, or back. In certain embodiments, the tissue protection layer may comprise a sensor configured to measure pressures in and around the wound. For example, the sensor may be used to measure the level of negative pressure applied to the wound or to measure the pressure on the underlying organs beneath the abdominal wound.

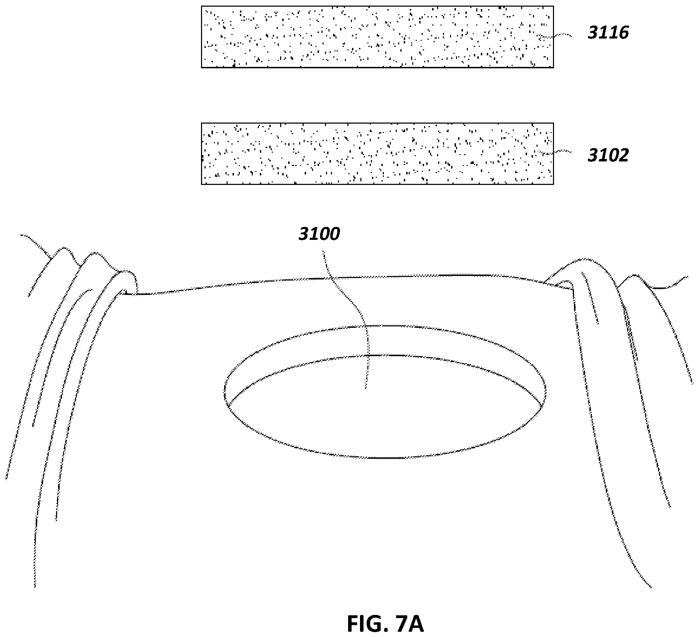

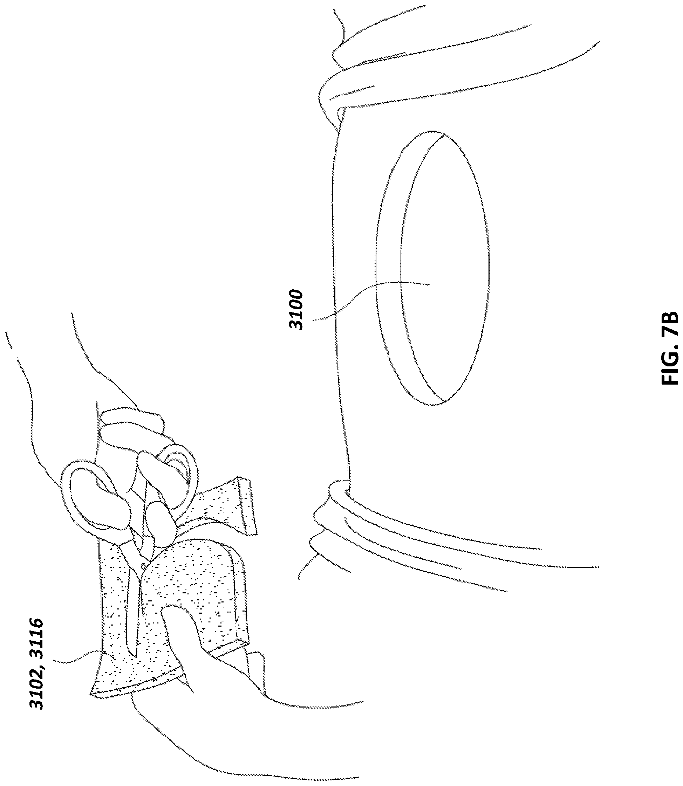

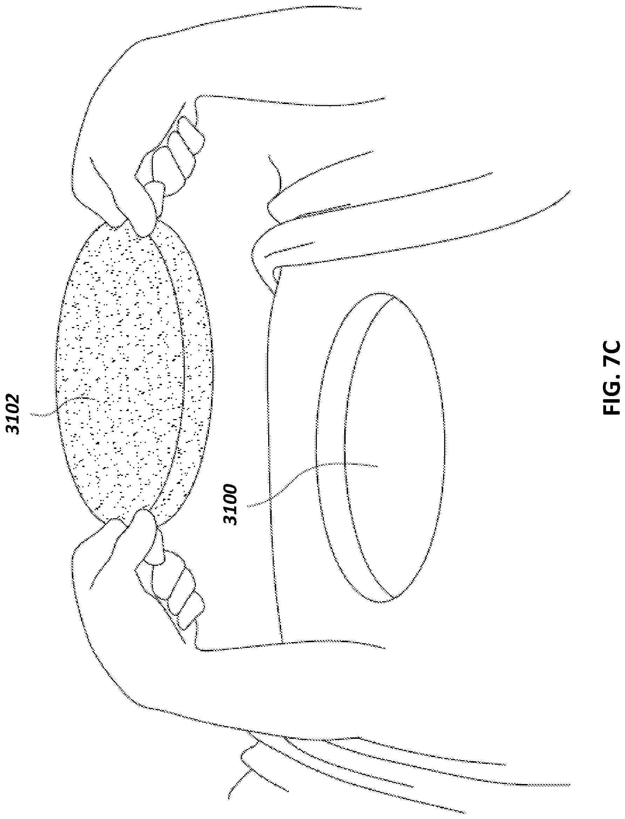

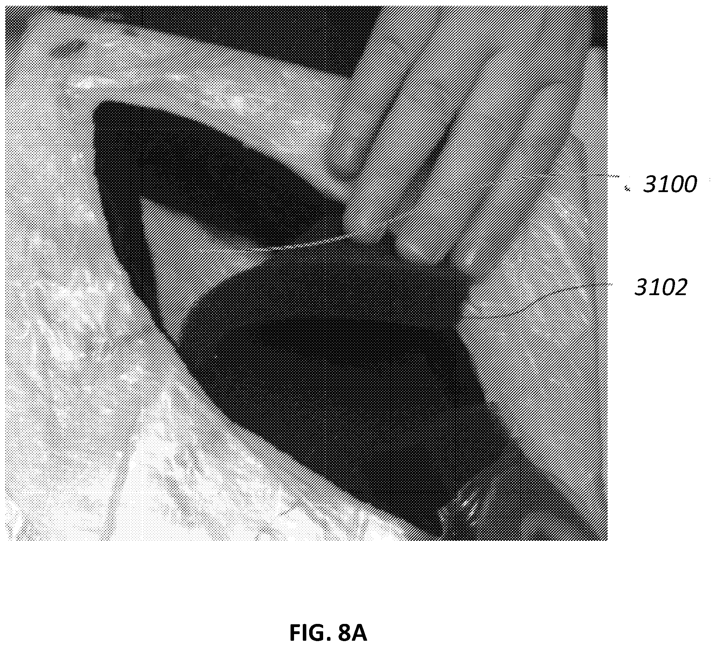

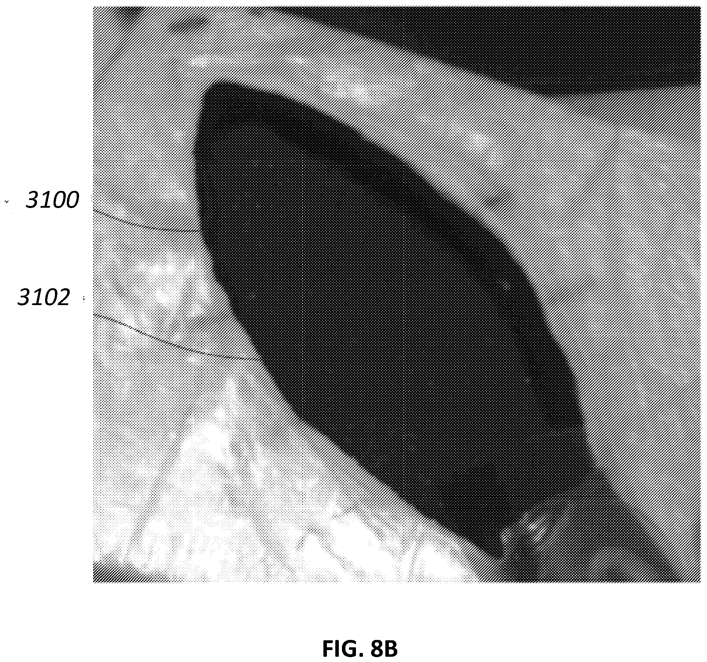

Figures 7A-C illustrate embodiments of possible initial steps in a method for the treatment of an open wound. However, as described above, the steps need not be performed in this order and may be performed in any order. In Figure 7A, two pieces of a porous material such as foam, a bottom piece 31 02 and a top piece 3116 are selected so as to approximate the size of the wound 3100. In some embodiments, the top piece and the bottom piece are of identical thickness. However, in certain embodiments, and vice-versa, top piece 3116 may be at least twice as thick, at least four times as thick, at least 10 times as thick or more than ten times as thick as bottom piece 3102. Figure 7B illustrates an embodiment of additional steps in a method for the treatment of an open wound. Bottom piece 3102 may be shaped via cutting or other suitable means to the shape of the wound and subsequently placed into the wound 3100, as shown in Figure 7C and depicted further below in Figure 8A.

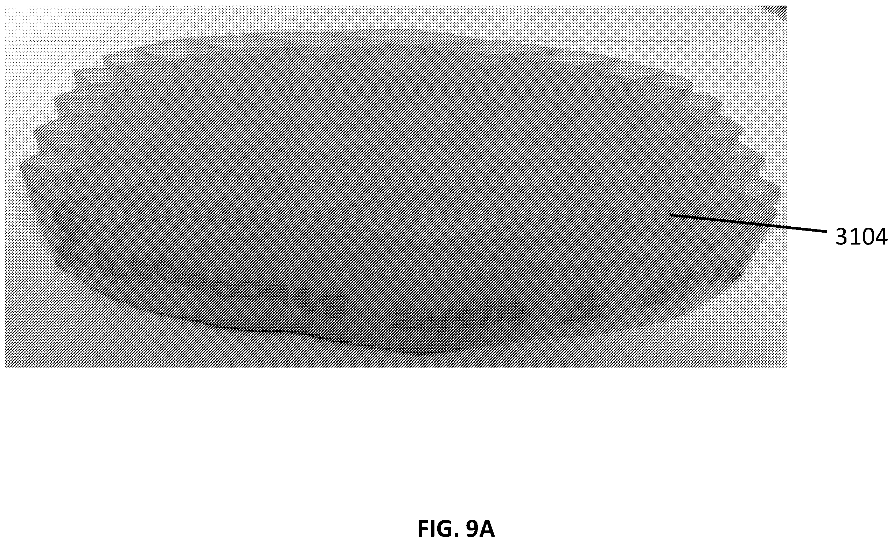

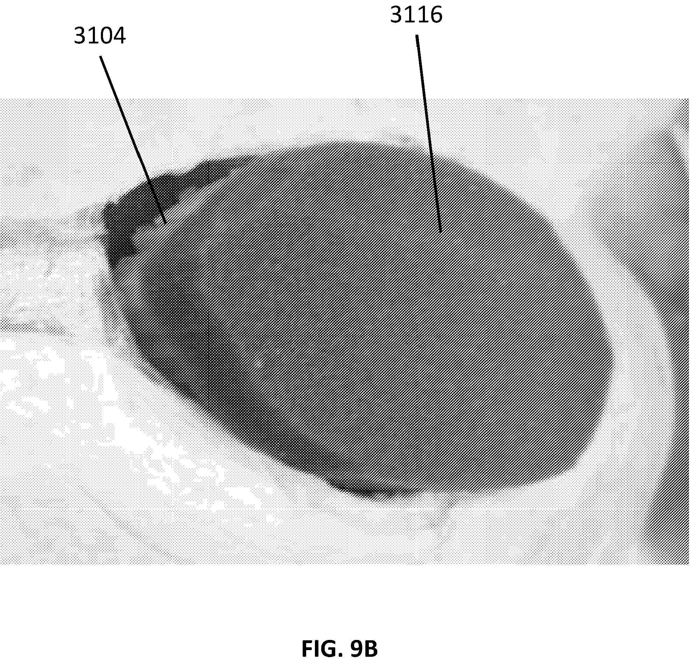

Figures 8A-B are photographs of a foam layer 3102 (for example, a 15 mm layer of foam), after shaping, placed into a wound bed 3100. In Figures 9A-C, a stabilizing structure 3104 similar to the stabilizing structures disclosed in Figures 2A-3E or any other stabilizing structure described elsewhere in the specification, is in the shape of the wound. The stabilizing structure may be shaped into the shape of the wound via cutting or other suitable means or the stabilizing structure may initially be of a size that is readily accommodated by the wound. As displayed in Figure 9B, the stabilizing structure 3104 may be placed into the wound. To assist with the insertion of the device into the wound bed, the device can be deformed slightly inwardly or horizontally to facilitate entrance into the wound site. In some embodiments, the device may be squeezed slightly during insertion and then release upon contact with the walls of the wound. In certain embodiments, the wound closure device 3104 may be placed such that the longitudinal sides of the matrix align with the longitudinal axis of the wound 3100. Continuing with Figure 9B, another foam layer 3116 (for example, a 10 mm layer of foam) is placed on top of the wound closure device 3104.

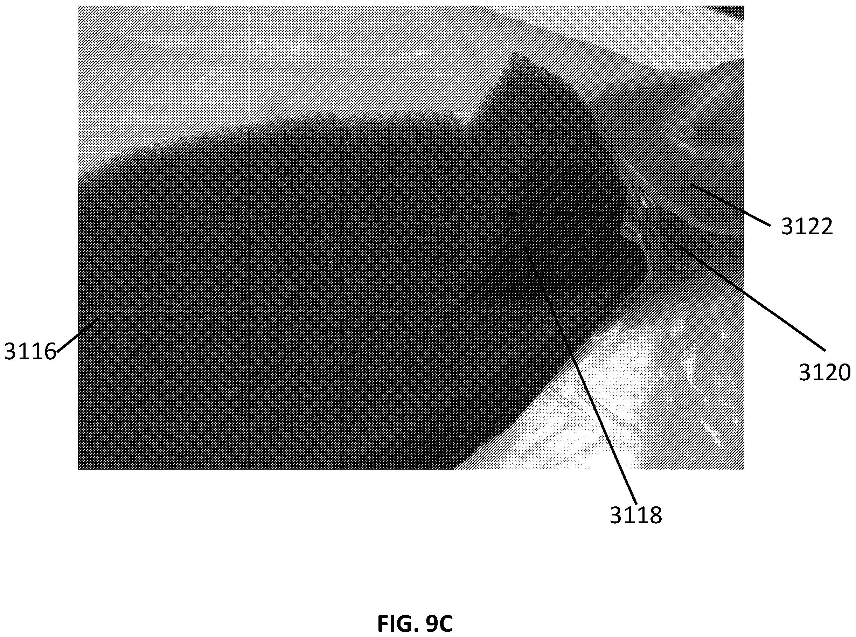

Figure 9C is a photograph of application of a port 3122 to the stabilizing structure and foam of Figures 9A-B. A bridging portion of foam 3118 may be placed in intimate contact with the foam layer 3116 at the edge of the wound. The bridging portion of foam 3118 may extend over intact skin, with a piece of drape 3120 placed between it and the intact skin. Further, a suction port 3122 may be connected to the bridging portion 3118 with a section of drape 3120 between. In alternative embodiments, the bridging portion 3118 and suction port 3122 may be placed on the wound during a different step depicted in Figures 8A-9B.

In Figure 10, as shown by steps 1-4, the device may be covered by one or more drapes 3120. A hole may be made in the drape covering the bridging portion of foam, and a suction port 3122 may be placed over the hole. A protective layer 3124 on the top surface of the one or more drapes may be removed after the drapes 3120 are applied. Once the drapes 3120 are applied and the port is in place, negative pressure may be applied to the wound through the drape from a vacuum source. The negative pressure can cause the stabilizing structure to collapse horizontally as described elsewhere in this specification. The tissue anchors adhered to the stabilizing structure through the porous layer engage tissue of the wound and may facilitate closure of the wound.

In certain embodiments, the suction port may be placed directly over the central portion of the foam layer 3116. In such embodiments, the foam layer may collapse inward along with the stabilizing structure while under negative pressure, thereby collapsing the suction port. To avoid collapse, the suction port may be rigid in comparison to the foam and resist collapse. A washer may be placed inside, below, or around the suction port to provide rigidity and resist collapse.

In some embodiments, the suction port may be pre-attached to the top foam layer so that drapes can be positioned around the port. A hard port or a soft port may be used, such ports may further be used in combination with a washer such as described above. In further embodiments, the suction port could only partially collapse with the collapsing matrix while still maintaining the port opening for negative pressure.

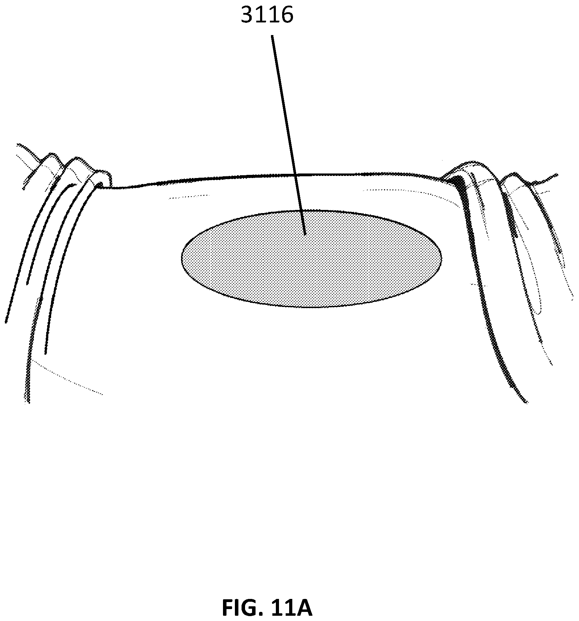

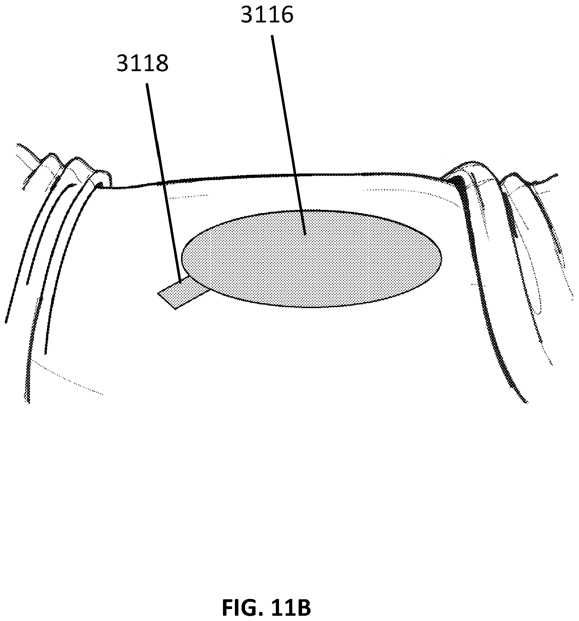

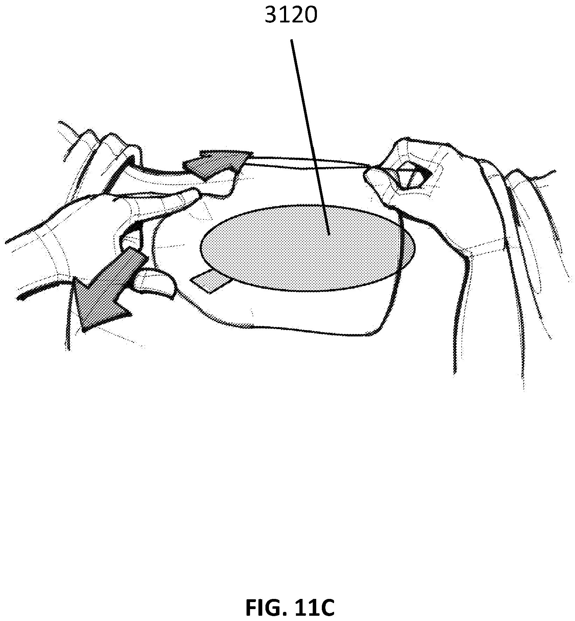

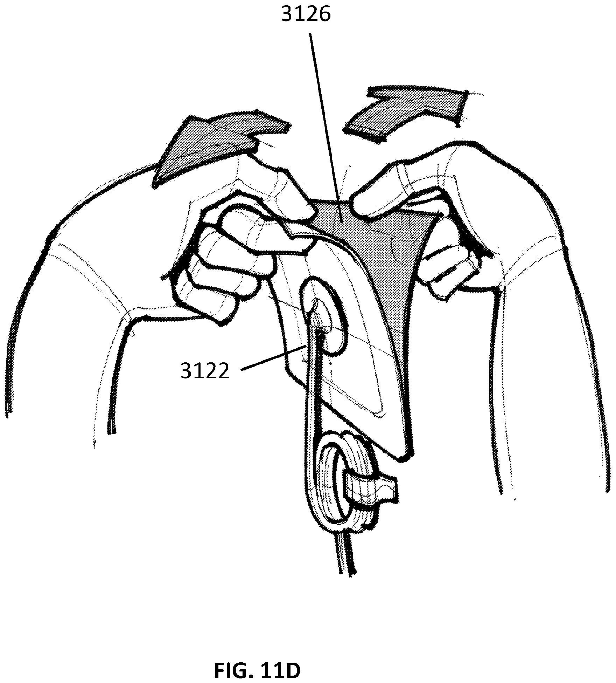

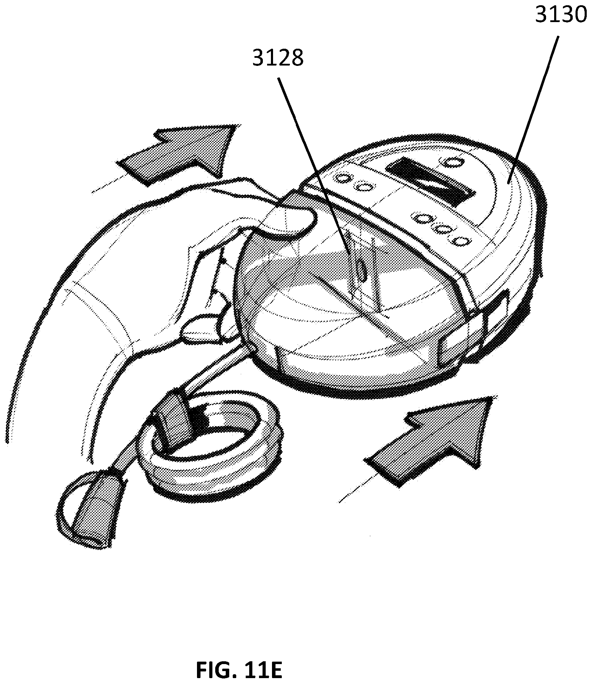

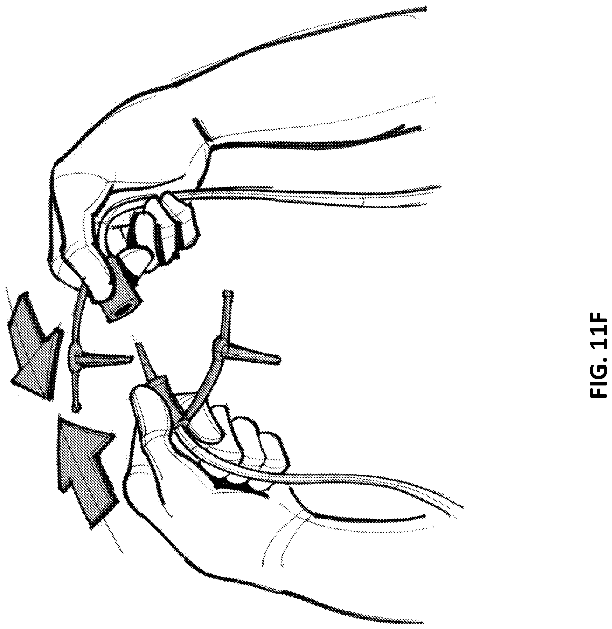

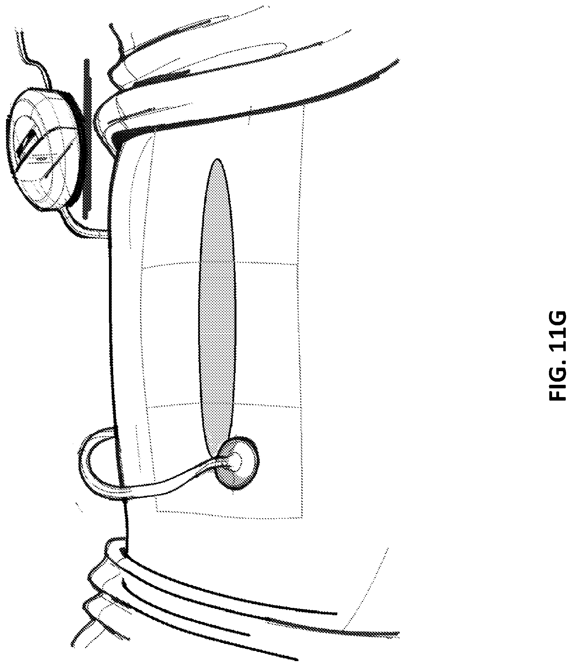

Figures 11A-11C provide further illustrations of an upper foam layer 3116 being placed in a wound, followed by placing a bridging portion 3118 and placing one or more drapes or wound covers 3120. Figures 11D-11G illustrate an embodiment of several steps in a method for the treatment and closure of a wound. As illustrated in Figure 11D, a suction port 3122 is separated from a release liner 3126 and later applied to a wound as depicted in Figures 8A-10. Figure 11E illustrates a canister 3128 being inserted into a negative pressure wound therapy device 3130 in preparation for the collection of wound exudate. Figure 11F illustrates the snap connection between the tubing connected to the suction port and the tubing connected to the negative pressure wound therapy device 3130. Once the connection has been made, negative pressure wound treatment may begin as depicted in Figure 11G.

Further details regarding the wound closure devices, stabilizing structures, related apparatuses and methods of use that may be combined with or incorporated into any of the embodiments described herein are found elsewhere throughout this specification and in International Application No. PCT/US2013/050698, filed July 16, 2013, published as WO 2014/014922 A1.

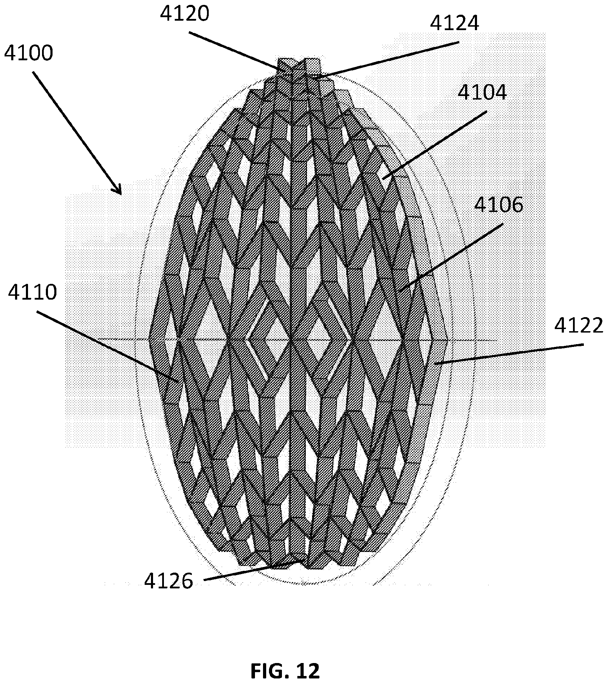

### The Stabilizing Structures of Figures 12-13C

Figure 12 is a drawing of an embodiment of a stabilizing structure 4100, similar to the stabilizing structures of Figures 2A-3E. Stabilizing structure 4100 may be constructed via any means described herein this section or elsewhere in the specification, such as via 3D printing and via the calculation method described in Figures 3A-3E. Further, stabilizing structure 4100 may be constructed from any material described herein this section or elsewhere in this specification such as the materials described in relation to Figures 2A-3E. Similar to the stabilizing structures of Figures 2A-3E, stabilizing structure 4100 comprises a plurality of elongate strips 4106 arranged in parallel or semi-parallel, whose longitudinal length can be aligned with the longitudinal axis of a wound. In embodiments, the elongate strips 4106 may also be arranged in a non-parallel fashion. The various cells within this stabilizing structure 4100 may have a variety of shapes and sizes. As was described in greater detail above, the length and shape of the elongate strips 4106, intervening members 4110, and cells 4104 may be designed so as to facilitate greater closure of the stabilizing structure.

In embodiments, the stabilizing structure of Figure 12 differs from the stabilizing structures of Figures 2A-3E, due to the inclusion of an extended section 4120. Extended section 4120 comprises one or more additional cells that extend outward along the longitudinal axis of the stabilizing structure 4100. Extended section 4120 may allow the stabilizing structure to better fit within a long incisional wound. Further, the addition of extended section 4120 may serve to prevent pinching of the surrounding tissue during collapse of the stabilizing structure 4100. Extended section may comprise about 6 additional cell, 12 additional cells, 16 additional cells, 20 additional cells, 30 additional cells, or more than 30 additional cells.

As depicted in Figure 12, extended section 4120 may include additional rows having progressively fewer cells across its width. For example, extended section 4120 may comprise a row of four cells, then a row of two cells, followed by another row of two cells. In some embodiments, a row of six cells precedes the row of four cells. The extended section 4120 extends beyond the outer edge of a virtual ellipse formed by the majority of the perimeter of the stabilizing structure along the longitudinal axis of the stabilizing structure. In certain embodiments, the extended section may extend from both ends of the stabilizing structure along the longitudinal axis. The extended section 4120 in some embodiments provides a stepped outer perimeter to the outer wall of the stabilizing structure at the longitudinal edges of the stabilizing structure, in contrast to the continuous outer perimeter along the sides of the stabilizing structure 4122.

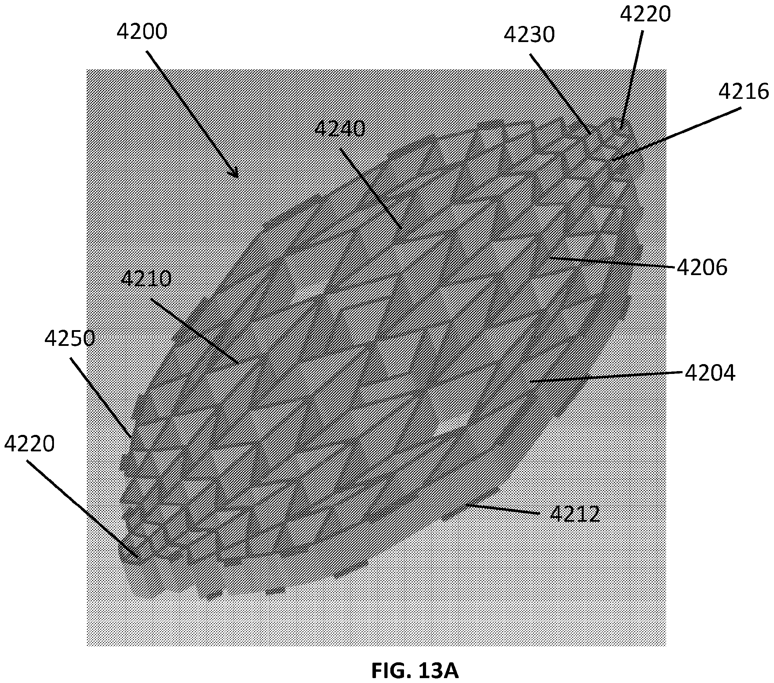

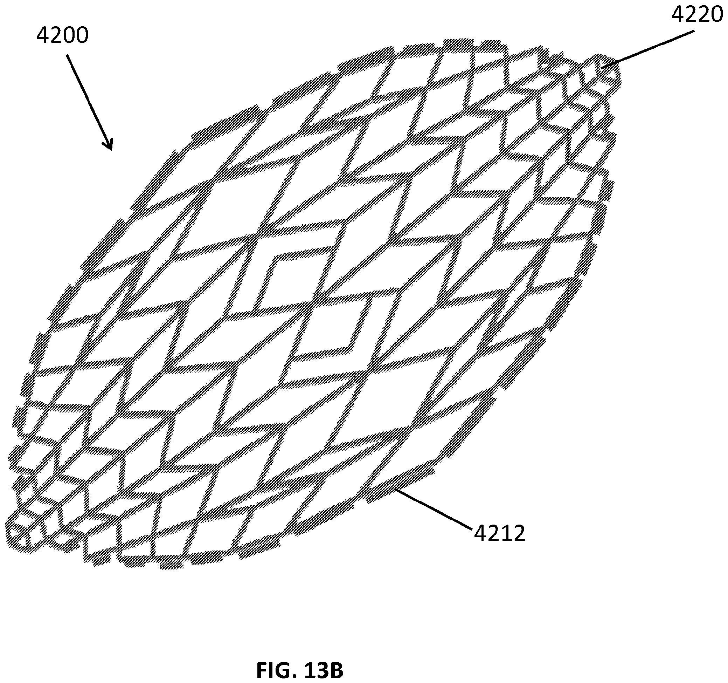

Absent the extended section 4120, the stabilizing structure comprises non-stepped side walls along substantially the entire length of the oval. However, with the extended section, the additional rows may provide a stepped outer perimeter 4124 based on the additional rows, in contrast to the flattened oval end of the stabilizing structure 4126. Further embodiments of the extended section will be described in more detail below in relation to Figures 13A-13C.

In some embodiments, the stabilizing structure may be in the form of two partial ellipse portions, elliptiforms, which are mirror images over a centerline of the stabilizing structure.

Figures 13A-13C are drawings of embodiments of stabilizing structure 4200, similar to the stabilizing structures of Figures 2A-3E and Figure 12. Much like the stabilizing structures disclosed elsewhere in the specification, stabilizing structure 4200 comprises elongate strips 4206, cells 4204, and intervening members 4210. Stabilizing structure 4200 further comprises extended sections 4220 at both ends of the longitudinal axis of the stabilizing structure. As described above in relation to Figure 12, extended sections 4220 may allow the stabilizing structure to better fit within the contours of a wound. Further, extended sections 4220 may prevent pinching of the surrounding tissue after collapse of the stabilizing structure. As described above, extended section may comprise multiple cells.

The stabilizing structures of Figures 13A-13C, and any of stabilizing structure disclosed herein this section or elsewhere in the specification may be produced in a variety of sizes. The possible size and shape of an actual wound may vary dramatically in size and shape, thus suitable stabilizing structures may also be prepared in a variety of sizes. For example, the length of an un-collapsed stabilizing structure may be approximately at least 25 mm, 50 mm, 75 mm, 100 mm, 125 mm, 150 mm, 1 75 mm, 200 mm, 250 mm, 300 mm, 350 mm, 400mm, 450 mm, 500mm, 750 mm, or greater than 750 mm. In certain embodiments, the width of an un-collapsed stabilizing structure may be at least 10mm, 15 mm, 25 mm, 35 mm, 50 mm, 75 mm, 100 mm, 125 mm, 150 mm, 175 mm, 200 mm, 250 mm, 300 mm, 350 mm, 400mm, 450 mm, 500mm or greater than 500 mm.

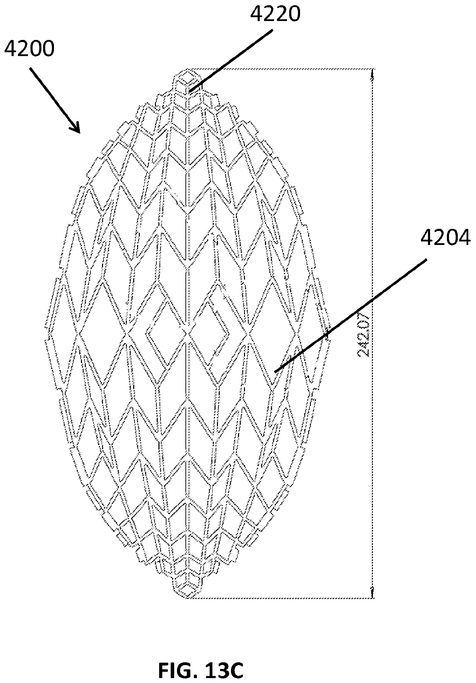

As depicted in Figure 13C, in some embodiments the un-collapsed stabilizing structure may have a length of approximately 242 mm. However, the stabilizing structure may be of any size disclosed herein this section or elsewhere in the specification. The cells 4204 of the stabilizing structure may be of a variety of sizes, for example the width of a cell 4204 may be approximately at least 5 mm, 10 mm, 15 mm, 20 mm, 25 mm, 30 mm, 50 mm, or more than 50 mm. For example, the length of a cell may be approximately at least 5 mm, 10 mm, 15 mm, 20 mm, 2 5 mm, 30 mm, 50 mm, or more than 50 mm.

In some embodiments, extended sections 4220 may comprise a first row of four cells, followed by a row of two cells, followed by another row of two cells. The row of four cells may be preceded by a row of six cells. However, in further embodiments, the extended section may comprise various numbers of cells per row and different numbers of rows. For example, extended section may comprise 1 row, 2 rows, 3 rows, 4 rows, 5 rows, 6 rows, or more than 6 rows. In embodiments, the rows may comprise 1 cell, 2 cells, 3 cells, 4 cells, 5 cells, 6 cells, 8 cells, 10 cells, 16 cells, or more than 16 cells.

Returning to Figure 13A, in certain embodiments, the extended section may comprise a series of cells 4104 comprising walls that are semi-parallel 4230 to the longitudinal axis of the stabilizing structure. These cell walls contrast with cell walls elsewhere in the stabilizing structure which comprise walls that run at an angle 4240 to the longitudinal axis of the stabilizing structure 4200.

In embodiments of the stabilizing structure comprising extended sections 4220, elongate members 4206 closest to the central longitudinal axis of the stabilizing structure extend further along the longitudinal axis than embodiments of the stabilizing structure that do not comprise an extended section. For example, the innermost elongate strips are the longest strips, while the next innermost strips are the second longest and so on. The presence of the extended sections causes the stabilizing structure when viewed from above to appear to be more eye-shaped rather than more oval-shaped.

As depicted in Figure 13A-C, in embodiments, the stabilizing structure 4200 may be oculiform. An oculiform shape may appear to be shaped like a human eye, with curved upper and lower edges converging to points at either longitudinal pole in the corners of the eye. Here, the outer walls curve inward 4250 to converge at the extended sections 4220. This shape is in contrast to a more diamond shape (not shown) where the outer walls would converge in a straight line to extended section 4220. However, in some embodiments, the stabilizing structure may be in the form of a diamond, rather than an oculiform.

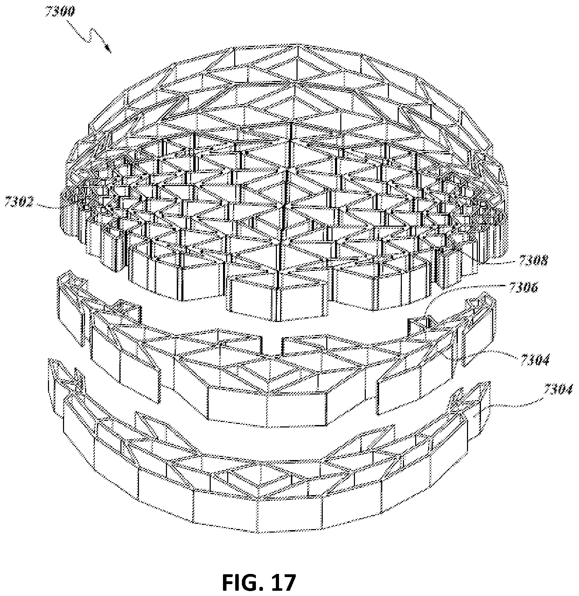

Stabilizing structure 4200 further comprises tabs 4212 extended outward from the outer wall of the stabilizing structure 4200. Such tabs may extend outward from the top or the bottom of the stabilizing structure or both. The tabs may extend out from all outer cells of the stabilizing structure as depicted by Figure 17B or the tabs may alternate as depicted in Figure 17A. The tabs may be constructed from any material described herein this section or elsewhere in the specification, such as those materials used for construction of the stabilizing structures. In certain embodiments, the tabs may be 3D printed as part of the stabilizing structure.

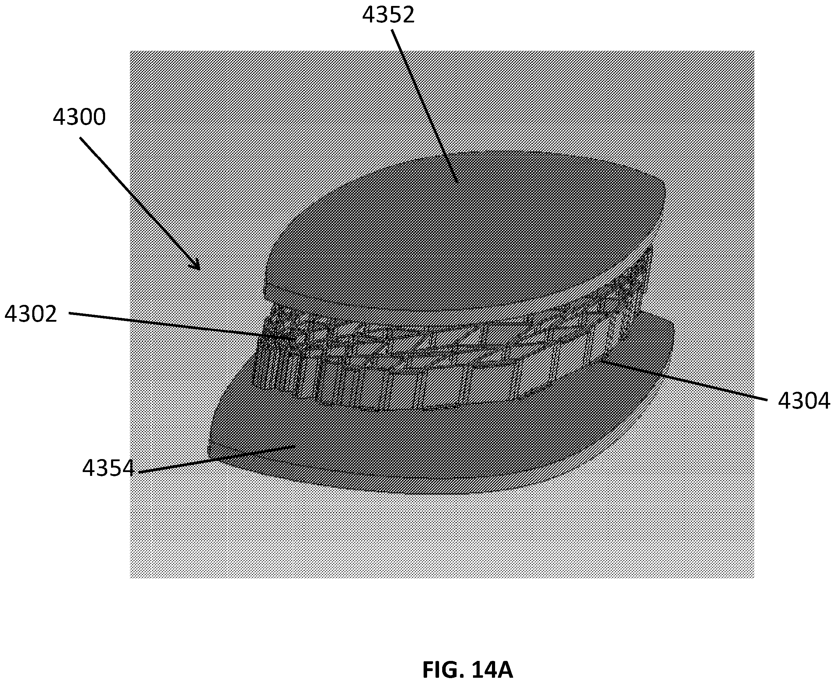

The tabs 4212 may further comprise an anchoring layer, which may be used to adhere the tabs to a layer of foam. In embodiments, the tabs may be coated in a suitable adhesive, allowing the tabs to be adhered to a layer of foam. The attachment of foam to the upper and lower layers of the stabilizing structure will be described in greater detail below in relation to Figure 14A-14D. The tabs may further serve to extend outward above or below tissues surrounding the stabilizing structure or around other structures such as foam, wrapped around the perimeter of the stabilizing structure.

The stabilizing structures of Figures 13A-13C may be provided in a variety of sizes such as those described above in relation to Figures 2A-3E. As described above, it may be advantageous in a clinical setting to minimize adjustments to the size of the stabilizing structure, therefore a kit may be provided that includes stabilizing structures of various sizes that may be fit to a wound of the appropriate size. For example, the kit may comprise only two sizes of matrices, a large size and a small size. The larger size stabilizing structure may be at least about 1.25x, 1.5x, 1.75x, 2x, 2.5x, 3x, 4x, 5x, 6x or greater than 6 times the size of the smaller stabilizing structure.

### The Stabilizing Structures and Foam Layers of Figures 14A-14D

Figures 14A-14D are drawings and photographs of foam layers in combination with stabilizing structures such as those described above in relation to Figures 2A-3E and 12-13C. The foam layers described below may include any type of foam described herein this section or elsewhere in the specification. Possible foams may include open-celled and/or reticulated foams made from a polymer. Suitable foams include foams composed of, for example, polyurethane, silicone, hydrophobic materials, hydrophilic materials, open-celled materials, close-celled materials, mixed open and close-celled materials, reticulated materials, polyester, silicone, and/or polyvinyl alcohol. In embodiments, the foam layers described herein may include materials that change their properties over time. For example, a particular foam may be rigid initially but become more flexible when wet and/or lose rigidity over time due to degradation of the material.

The foam layers described in this section or elsewhere in the specification may have a variety of suitable thicknesses. For example, a foam layer may have a thickness of at least about 1 mm, 3 mm, 5mm, 10 mm, 15 mm, 20 mm, 25 mm, 30 mm, 35 mm, 40 mm, 45 mm, 50 mm, or more than 50 mm thick. Single layers of foam may be laid atop one another to create a greater total thickness of foam, for example, a 15 mm thick layer of foam may be laid atop a 10 mm layer of foam to create a 25 mm total thickness of foam.

In certain embodiments, any of the foam layers described herein this section or elsewhere in the specification, may be pre-attached to an organ protection layer such as described above. For example, the lowest layer of foam, closest to the underlying organs, may be attached to an organ protection layer before placement within the wound, thereby saving the clinician the step of first placing an organ protection layer within the wound. In certain embodiments, the organ protection layer may be pre-attached to the underside of a stabilizing structure such as those described herein this section or elsewhere in the specification. In embodiments, the organ protection layer may be attached to the top of the bottom-most foam layer placed in the wound, thereby positioning the organ protection layer between the stabilizing structure and the bottom-most layer of foam. The organ protection layer may completely encase the bottommost layer of foam or stabilizing structure. The presence of a bottom layer of foam and/or organ protection layer may serve to protect the underlying bowel from damage due to direct interaction with the stabilizing structure.

Figures 14A-C are drawings and photographs of embodiments of a wound closure device 4300 comprising a stabilizing structure 4302 (similar to the stabilizing structures described above in relation to Figures 2A-3E and 12-13C), a top porous foam layer 4352, and a bottom porous foam layer 4354. As will be described in greater detail below, top and bottom porous layers 4352 and 4354 may be shaped in any desired manner to conform to the shape of stabilizing structure 4302. In embodiments, the top and bottom layers of foam may be attached to the stabilizing structure 4302 before placement in the wound. Pre-attachment of the foam layers advantageously reduces the number of steps that need to be completed by the clinician

As described elsewhere in the specification, stabilizing structure 4302 may comprise tabs 4304. These tabs advantageously provide a larger surface area for attachment of the foam layers to the stabilizing structure. Without the tabs, adhesive would necessarily need to be applied to the narrow upper edges of the stabilizing structure, potentially creating a weak or non-existent attachment. As described above, the tabs may be located on the top and bottom edges of the stabilizing structure. In embodiments, rather than adhesive, the tabs may be covered in anchors, which may act much like the adhesive, allowing the foam layers to be attached to the stabilizing structure prior to placement in the wound. The stabilizing structure may be pre-attached to the bottom layer of foam, top layer, or both. In certain embodiments, the adhesive may be applied to the central longitudinal elongate member of the stabilizing structure rather than to the tabs or other location. By applying adhesive only to the central elongate member, the stabilizing structure may collapse without resistance from the foam.

Figures 14A-C show embodiments of wound closure devices where the bottom foam is larger than the top foam, either by width, length, or both. Here, the foam extends outward from the stabilizing structure to create a lip, thereby allowing the lip of foam to extend above or below the surrounding tissue layers such as the fascia. The lip may serve to maintain the stabilizing structure in place by providing a downward force to resist the upward force applied by the expanding underlying viscera. In certain embodiments, the lip may need to be folded during placement within the wound bed so as to allow the closure device to be properly positioned. Thereafter the lip may unfold and extend into the surrounding tissues to aid in securing the device and applying negative pressure to the surrounding tissues.

In certain embodiments, the wound closure device of 4300 may be dome-shaped as described in more detail elsewhere in the specification. In certain embodiments, the stabilizing structure may be dome shaped and/or the bottom and or the top layer of foam may be dome shaped. The stabilizing structure may be shaped such that the upper surface is concave while the bottom surface is convex. In some embodiments, the upper surface of the stabilizing structure is convex while the lower surface is concave. Any of the layers of foam (the top, bottom, middle or further layers of foam) may comprise an upper surface that is concave and a bottom surface that is convex. In some embodiments, any of the layers of foam (the top, bottom, middle or further layers of foam) may comprise an upper surface that is convex and a bottom surface that is concave.

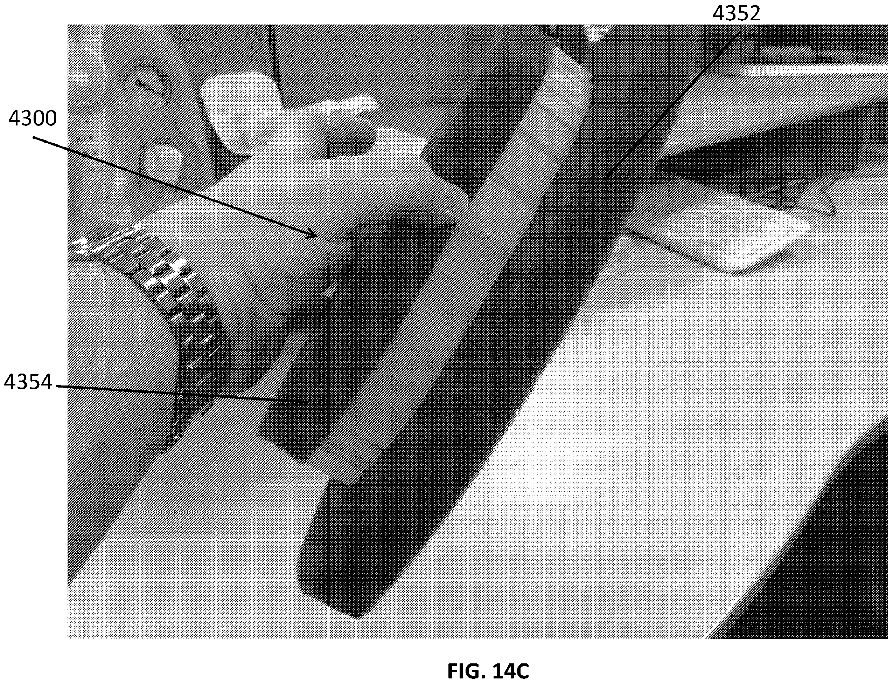

The top layer may be sized to the top of the stabilizing structure, thereby facilitating closure of the wound to the size of the collapsed stabilizing structure. The lip extending outward from the matrix may be rounded so as to provide a better fit within the wound. In contrast, in the embodiment of Figure 14C, the bottom layer may be smaller than the top layer. The top layer may advantageously prevent drawing of the drape down into the stabilizing structure or between the stabilizing structure and the edges of the wound.

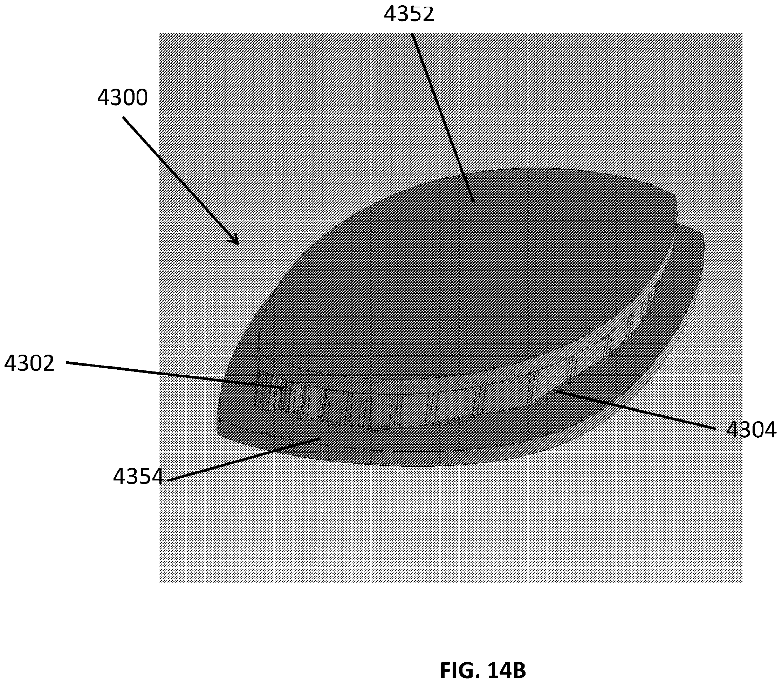

In certain embodiments, the foam layers may be of any thickness disclosed herein this section or elsewhere in the specification. The bottom layer of foam 4354 may be approximately 15mm thick or approximately 10mm thick. For example, the bottom foam 43 54 of Figure 14B may be thicker than the bottom foam of Figure 14B.

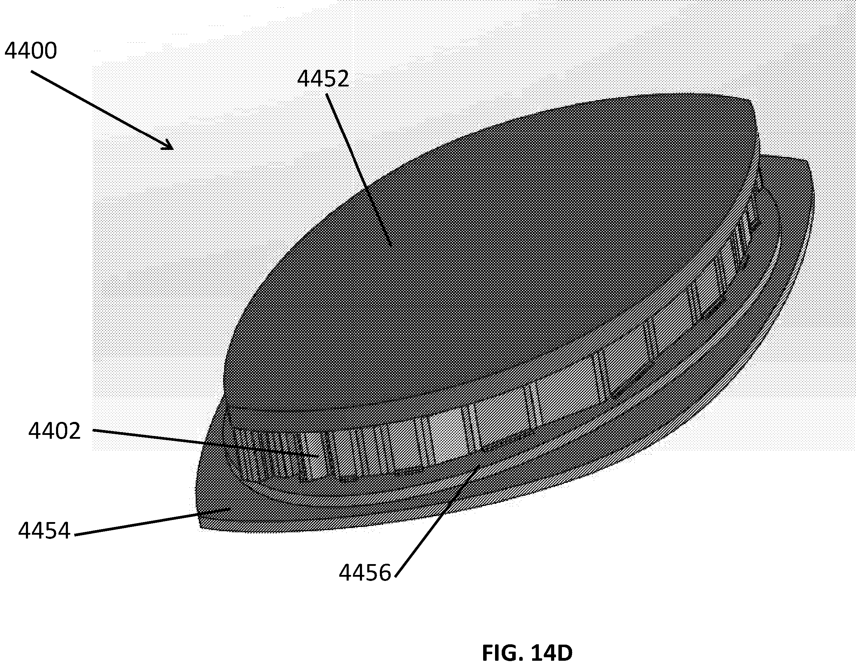

Figures 14D depicts an embodiment of a wound closure device 4400 involving a total of 3 layers of foam. Here, wound closure device 4400 comprises stabilizing structure 4402, top layer of foam 4452, bottom layer of foam 4454, and middle layer of foam 4456. The stabilizing structure may be pre-attached to the middle layer of foam, top layer of foam, or both. Further, the bottom layer of foam may be pre-attached to the middle layer of foam, or may be placed into the wound separately. In some embodiments, the top layer is 15 or 10 mm thick, the middle layer is 15 mm thick, and the bottom layer is 10 mm thick. Foam layers may be attached by any suitable means, such as via adhesive or anchors. As depicted in Figure 14D, the bottommost layer of foam may comprise a lip that extends outward from the wound closure device into the surrounding tissue. As described above, such a lip may secure the device in place. The bottom layer of foam may be wider and/or longer than the middle and/or top layers of foam. In certain embodiments, in addition to the foam on the top and bottom of the stabilizing structure, foam may be attached to the entire outer perimeter of the stabilizing structure. Foam may be attached to the perimeter of the stabilizing structure via any suitable means, such as by adhesive or anchoring layer. Once foam has been applied to the perimeter of the stabilizing structure, the stabilizing structure will no longer be visible if there are also top and bottom layers of foam.

In embodiments of the foam layers of Figures 14A-14D, the layers of foam may comprise any type of suitable foam material described herein this section or elsewhere in the specification. For example, the foam may comprise "black foam" such as polyurethane and/or "white foam" comprising polyvinyl alcohol (PVA). In embodiments involving PVA foam, thinner foam layers may be needed as compared to other types of foam, because PVA foam is often more resilient and dense than other types of foam. Further, once PVA foam becomes wet it may also aid with lateral slip. In some embodiments, the foam layers may be combined with other fillers such as gauze, or other mesh/net products such as those on Fry and Kossel.

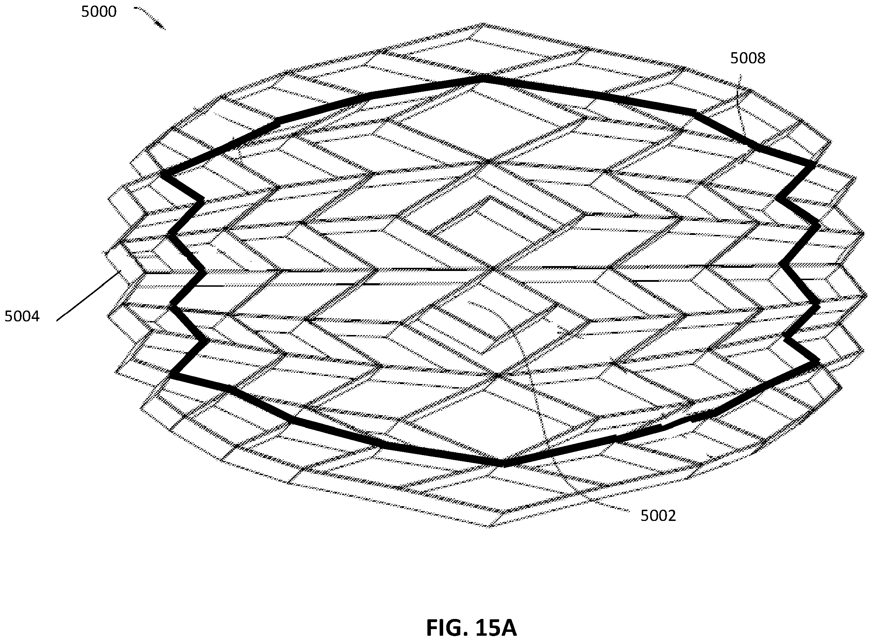

### The Wound Closure Systems and Apparatuses of Figures 15A - 19

In some instances, a stabilizing structure with adjustable size is desirable. For example, a stabilizing structure with adjustable size can accommodate to various sizes of wounds. Also, a single wound may change its size as the wound heals, and a stabilizing structure with adjustable size can be useful in such case. In some embodiments, a stabilizing structure may have a removable outer shell or detachable segments which may be removed to reduce the size of the stabilizing structure. Further, multiple outer shells and/or detachable segments may be removed to further reduce the size of the stabilizing structure.

Figures 15A-C depict embodiments of a stabilizing structure 5000 similar to the stabilizing structures disclosed in Figures 2A-C, 9A, 12, 13A-14D. Here, the stabilizing structure 5000 comprises an inner segment 5002 and an outer shell or detachable segment 5004, such that the outer shell 5004 may be removed to reduce the size of the stabilizing structure 5000.

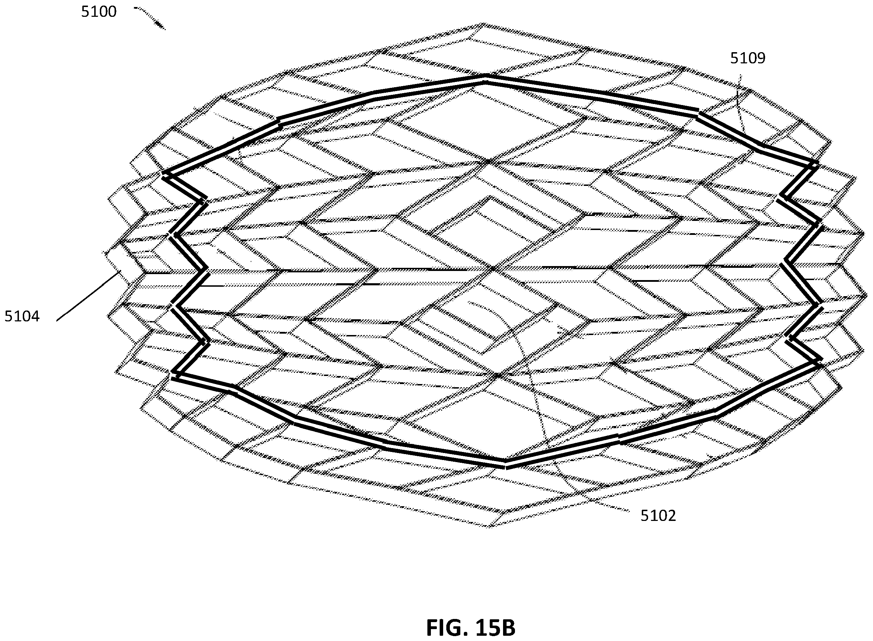

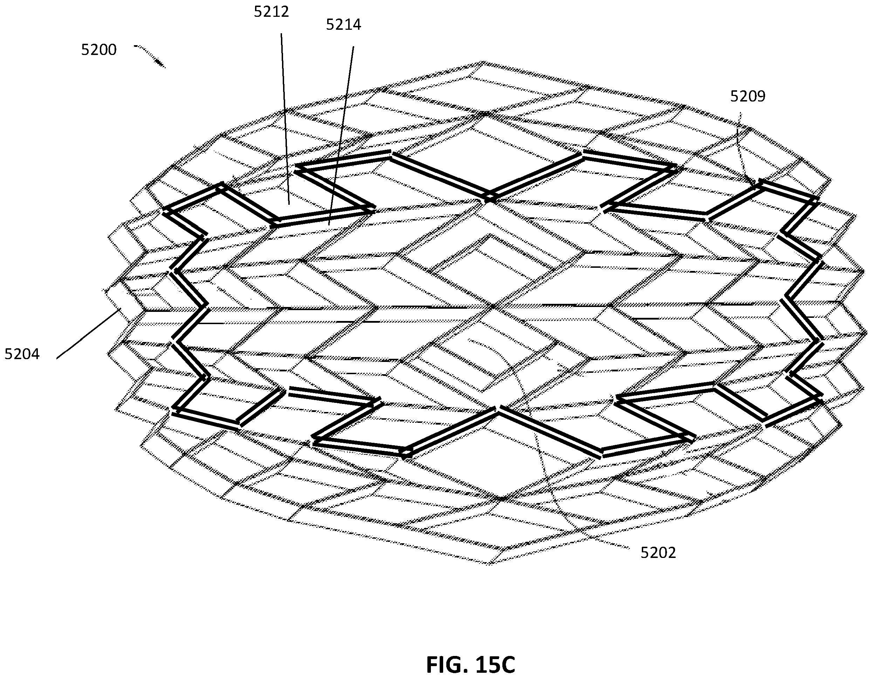

In some embodiments, as shown in Figure 15A, the stabilizing structure 5000 may comprise pre-cuts 5008 along the outline of the inner segment 5002, so that one or more outer shells 5004 are tearable from the inner segment 5002. In some embodiments, such as the stabilizing structure 5100 shown in Figure 15B, the outer shell 5104 and the inner segment 5102 closely interlock at the interface 5109, thereby allowing for ease of release without the need to cut the stabilizing structure. The outer shell 5104 may comprise attachment elements such as Velcro^{®}, adhesives, hooks, prongs or any other suitable means. In certain embodiments, the inner segment 5102 comprises receiving elements that is configured to receive the attachment elements of outer shells 5104. In some embodiments, the interface 5209 between the outer shell and the inner segment comprises at least partially jig-saw shape or crenellated shape as shown in Figure 15C. The extended cells 5212 of the outer shell may fit into the recesses 5214 of the inner segment, such that the outer shell 5204 can be separated from the inner segment 5202 by the application of force.

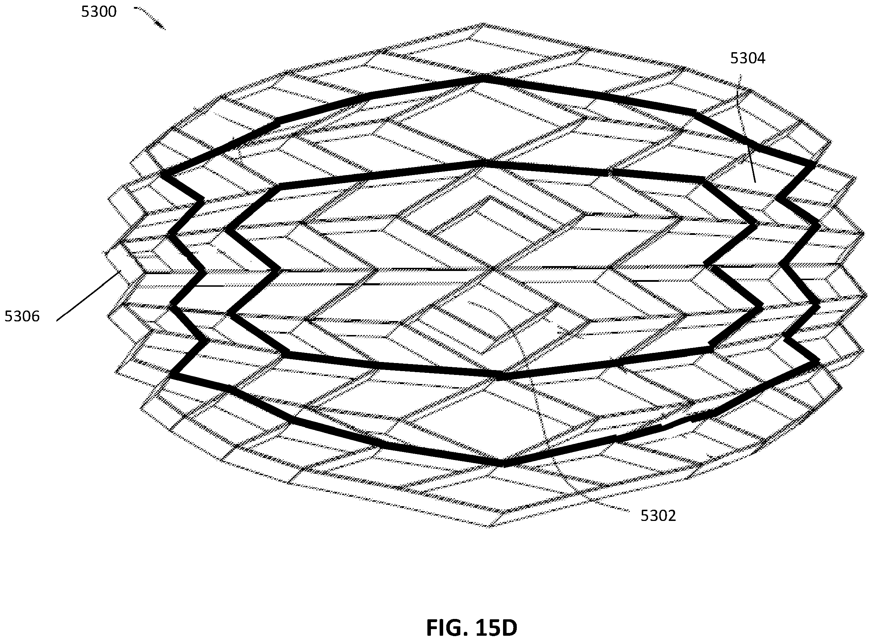

In some embodiments, multiple outer shells or detachable segments may be removed to further reduce the size of the stabilizing structure. For instance, Figure 15D depicts an embodiment of a stabilizing structure 5300 having an inner segment 5302, the first outer shell or detachable segment 5304, and a second outer shell or detachable segment 5306. In some embodiments, more than two outer shells or detachable segments may be removed. For example, there may be three outer shells, four outer shells, five outer shells, or more than five outer shells. In some embodiments, the outer shell may comprise multiple sections, for example, two sections on each side across the longitudinal axis of the stabilizing structure, so that each section can be removed selectively depending on size or shape of the wound. In some embodiments, the one or more outer shells are configured to be removed in a vertical direction. In some embodiments, the one or more outer shells are configured to be removed in a horizontal direction.

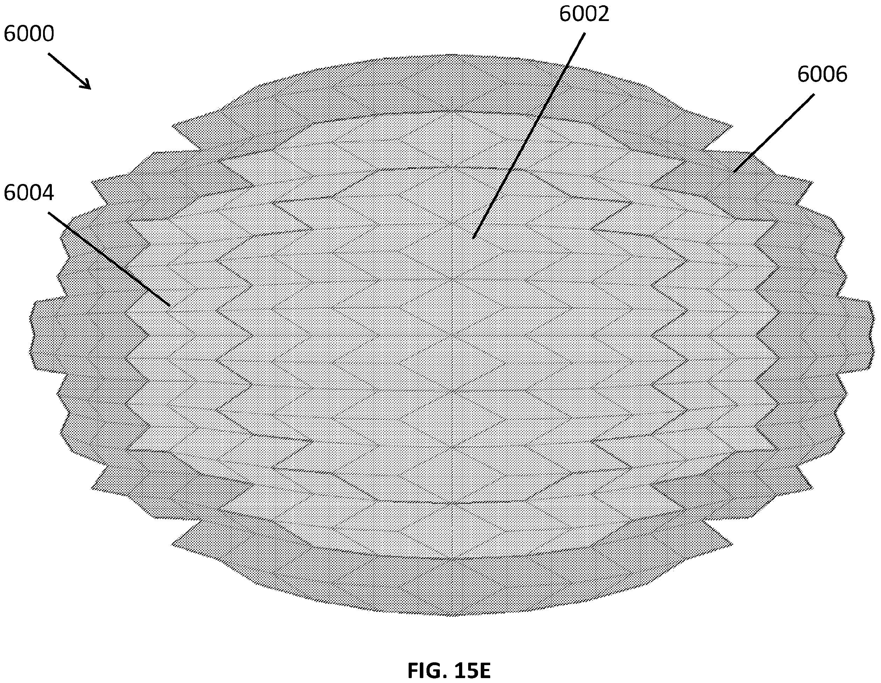

Figure 15E depict an embodiment of a stabilizing structure 6000, similar to the stabilizing structures disclosed previously in Figures 2A-2C, 9A, 12, 13A-14D. Here, the stabilizing structure 6000 comprises inner segment 6002, first outer shell or detachable segment 6004, and second outer shell or detachable segment 6006. In some embodiments, the first detachable segment 6004 at least partially surrounds or completely surrounds the inner segment 6002, and the second detachable segment or outer shell 6006 at least partially surrounds or completely surrounds the first detachable segment or outer shell 6004. In some embodiments, each of the segments may have an oculiform shape. To adjust the stabilizing structure to the shape of a wound, in embodiments, segments of the stabilizing structure 6002, 6004 may be removed from the overall structure to form a smaller stabilizing structure such as the inner segment 6002. In certain embodiments, detachable segments or outer shells may encompass the entire outer perimeter of the stabilizing structure, while in some embodiments, the segments may only comprise a small portion of the stabilizing structure. In certain embodiments, there may be at least: one, two, three, four, five, six, seven, eight, nine or ten detachable segments or outer shells. In some embodiments, the detachable segments or outer shells may comprise multiple sections, for example, two sections on each side across the longitudinal axis of the stabilizing structure, so that each section can be removed selectively depending on size or shape of the wound.

One of skill in the art will understand that the outer shells or detachable sections of the stabilizing structures of Figures 15A-18E, and any stabilizing structure and/or wound closure device disclosed herein this section or elsewhere in the specification, may be removed in any suitable direction. For example, the stabilizing structure may be configured such that the outer shell(s) or detachable section(s) may be removed horizontally within an x-y plane parallel to the longest dimension of the stabilizing structure. In certain embodiments, the stabilizing structure may be configured such that detachable sections may be removed in a vertical direction in the z axis, perpendicular to the x-y plane. The stabilizing structure may have at least one outer shell or detachable section removable in a horizontal direction and one shell or section removable in a vertical direction. The outer shell(s) or detachable section(s) may be attached to the stabilizing structure in such a manner that the outer shell(s) or detachable section(s) may only be removed in a single direction, such as by the use of slots and/or channels as the attachment and receiving elements.

In embodiments, the stabilizing structure segments may be cut from the stabilizing structure 6000 to produce a smaller structure. In certain embodiments, the stabilizing structure may have pre-cuts along the shape of the segments 6002, 6004 to allow the segments to be tearable and easily removed by hand from the stabilizing structure. The detachable segments may be adhered to the remainder of the stabilizing structure via adhesive, Velcro^{®}, or other suitable adhesive means. In certain embodiments, the removable sections may be held together by the tightness of the structures squeezing together and/or via friction. In some embodiments, magnets and/or suction cups may be used to keep the segments together.

As with the stabilizing structures depicted above in relation to Figures 14A - 14D, the stabilizing structure 5000, 5100, 5200, 5300, or 6000 may have a porous layer above the structure, below the structure, or both above and below the structure. In certain embodiments, the stabilizing structures may be attached to a porous layer, such as the upper layer and/or lower layer or layers. In such embodiments, the various segments of the stabilizing structure are not directly attached to one another; instead the segments are held together by a porous layer or layers attached to top and/or the bottom of the stabilizing structure. In embodiments, the porous layer(s) may have pre-cuts and/or perforations aligned with the detachable segments, thereby allowing a caregiver to tear portions of the foam and remove segments from the stabilizing structure along with the sections of foam. In some embodiments, the layer or layers of foam holding the stabilizing structure together may be positioned around the sides/perimeter of the stabilizing structure.

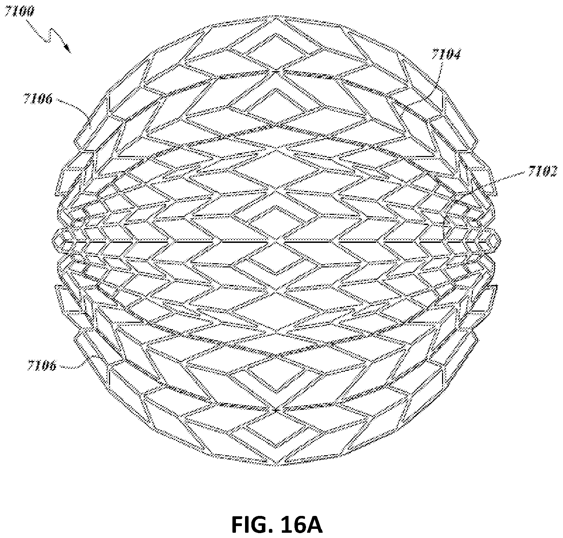

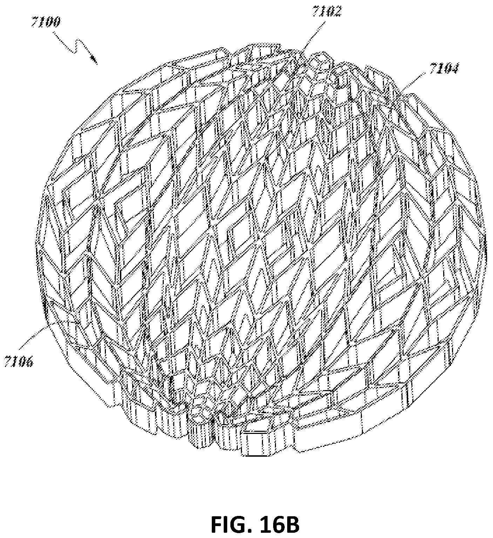

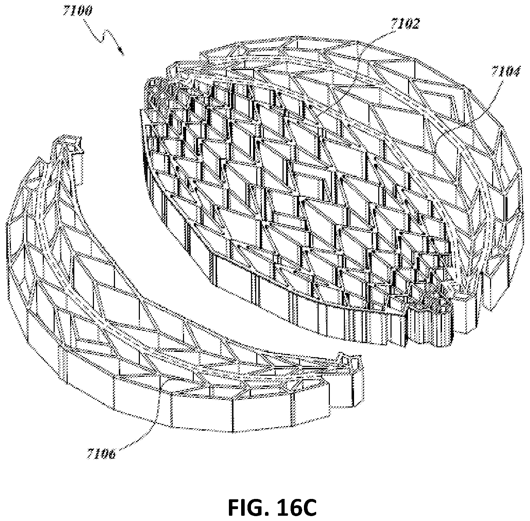

Figures 16A-B depict embodiments of stabilizing structures 7100 similar to the stabilizing structure of Figures 15A-D. As with the stabilizing structures of Figures 15A-D, stabilizing structure 7100 comprises detachable segments 7104 and 7106, and inner segment 7102. Here, the detachable segments may not extend around the entirety of the perimeter of inner segment. Instead, detachable segments 7104 and 7106 are split into two mirrored structures on either side of the inner segment 7102. As depicted in Figure 16C, multiple detachable segments may be removed at once.

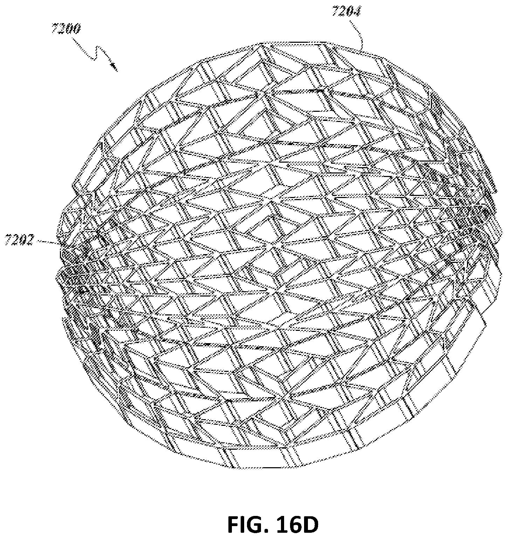

Figure 16D depicts an embodiment of a stabilizing structure 7200 where the detachable segments extend around the entirety of the perimeter of the inner segment. Here, the two mirrored sections of the detachable segments are linked 7206 at the end of the stabilizing structures.

Figure 17 depicts a stabilizing structure 7300 similar to the stabilizing structures of Figures 15A-16D. Here the detachable sections 7304 comprise extended cells 7306 which fit into recesses 7308 of inner segment 7302 or another detachable segment 7304. In embodiments, the extended cells are configured to snap fit into the recesses, such that the different segments can be separated from one another by the application of force. For example, separation can occur by the application of force by a caregiver.

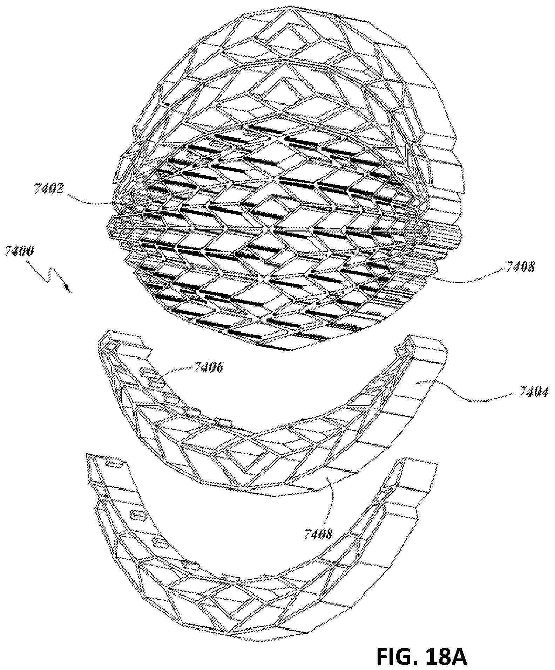

Figures 18A-18D depict embodiments of stabilizing structures 7400 similar to the stabilizing structures depicted in Figures 15A-17. Here, detachable segments 7404 comprise one or more attachment elements 7406, which may be in the form of prongs, hooks, tongues, screws, nails, or other suitable attachment means. The attachment elements 7406 attach to receiving elements 7408 which may be in the form of grooves, holes, windows, or any suitable means. The attachment elements serve to maintain attachment of the detachable segment to the inner segment or another detachable segment until the stabilizing element is re-sized by applying suitable force to separate the attachment elements from the receiving elements. In certain embodiments, detachable segments and inner segment may comprise both attachment elements and receiving elements. For example, a detachable segment may comprise attachment elements on one side and receiving elements on the opposite side to allow the detachable elements to be stacked one after another. In certain embodiments, segments may comprise at least 1, 2, 3, 4, 5, 6, 7, 8, 10, 15, 20, 30, or more than 30 attachment elements. In some embodiments, segments may comprise at least 1, 2, 3, 4, 5, 6, 7, 8, 10, 15, 20, 30, or more than 30 receiving elements.

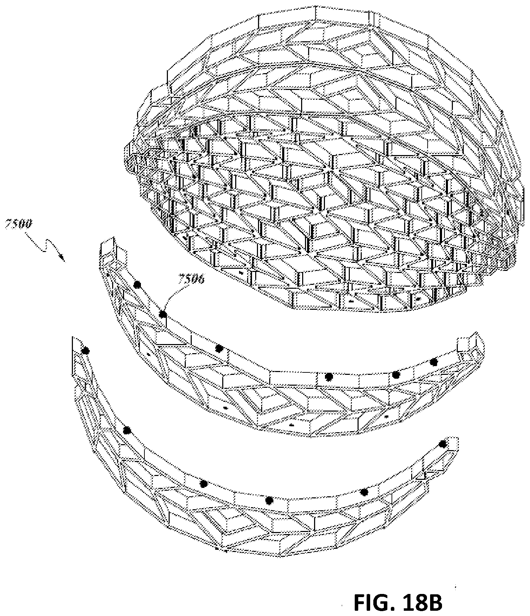

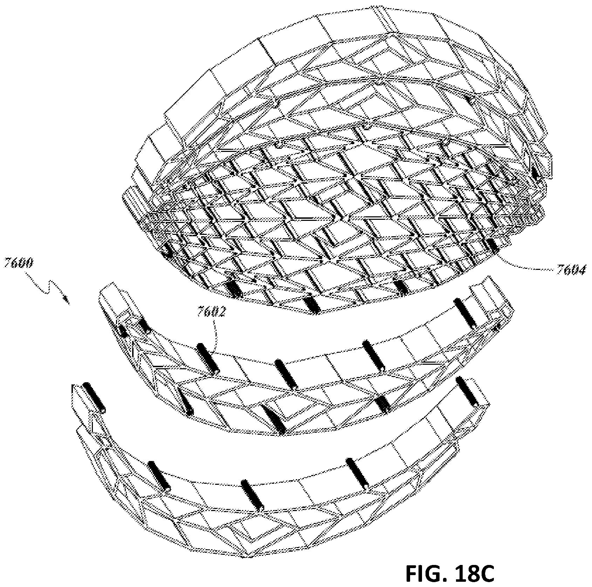

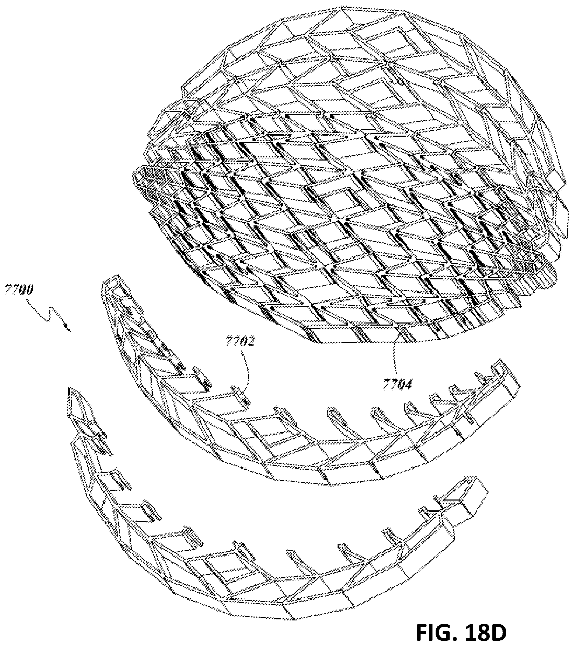

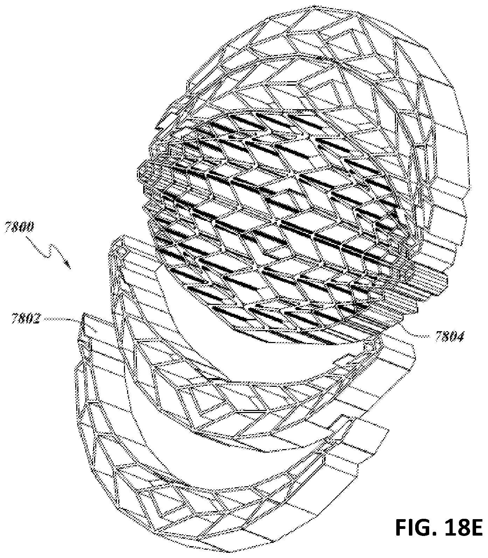

Figure 18B depicts an embodiment of a stabilizing structure 7500 where the attachment elements 7506 are prongs. Figure 18C depicts an embodiment of a stabilizing structure 7600 where the attachment elements 7602 are tongues which fit into the receiving elements, which are grooves 7604. Figure 18D depicts an embodiment of a stabilizing structure 7700 where the attachment elements 7702 are hooked and the receiving elements are configured to receive the hooks. Figure 18E depicts an embodiment of a stabilizing structure 7800 where adhesive may be applied to certain areas of the detachable segments for adhesion to the outer surfaces of other detachable segments or the inner segment. Adhesive may also be applied to the inner segment.

In certain embodiments, the detachable segments such as those disclosed above in relation to Figures 15A-18E may be packaged within a separate kit from the stabilizing structure, each of the detachable segments having a top foam, a bottom foam, both, or no foam at all. The separately packaged detachable segments may comprise attachment elements and/or receiving elements such as those disclosed herein this section or elsewhere in the specification. Such separately packaged detachable segments may then be added to main stabilizing structure to increase the size and/or alter the shape of the stabilizing structure. In certain embodiments, the separate kit(s) of detachable segments may contain one detachable segment, two detachable segments, three detachable segments, four detachable segments, five detachable segments, or more than five detachable segments. In some embodiments, the detachable segments may be in the form of a crescent.

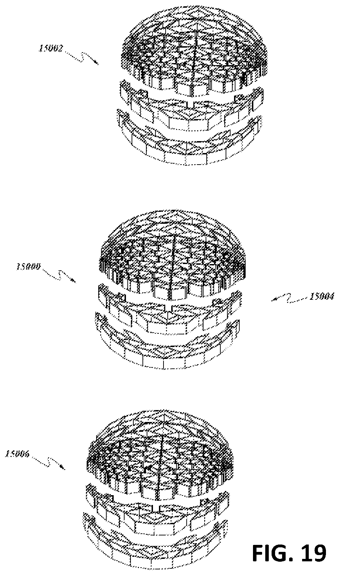

Figure 19 depicts an exploded view of wound closure device 15000, comprising stabilizing structures 15002, 15004, 15006 similar to those disclosed elsewhere in the specification, such as in relation to Figures 2A-3E, 12---18E. However, here, the stabilizing structures 15002, 15004, 15006 may be stacked one atop the other to provide a wound closure device 15000 with greater depth. Such increased depth may be advantageous in patients with additional abdominal tissue, such as those with increased adipose tissue, rendering a single stabilizing structure potentially inadequate. In some embodiments, the wound closure device may comprise two stackable stabilizing structures, three stackable stabilizing structures, four stackable stabilizing structures, five stackable stabilizing structures, or more than five stackable stabilizing structures. Similar to the detachable segments described above in Figure 15-18E, the stackable stabilizing structures may be packaged separately as kits. In embodiments, the separately packaged stackable stabilizing structures may have foam on a top and/or a bottom surface, thereby allowing there to be foam on the topmost and/or bottommost surfaces of the stacked stabilizing structures, and/or between the stacked stabilizing structures. In embodiments, the stackable stabilizing structures contain attachment elements and/or receiving elements, such as those disclosed herein this section or elsewhere in the specification, allowing the stackable stabilizing structures to be attached to one another.

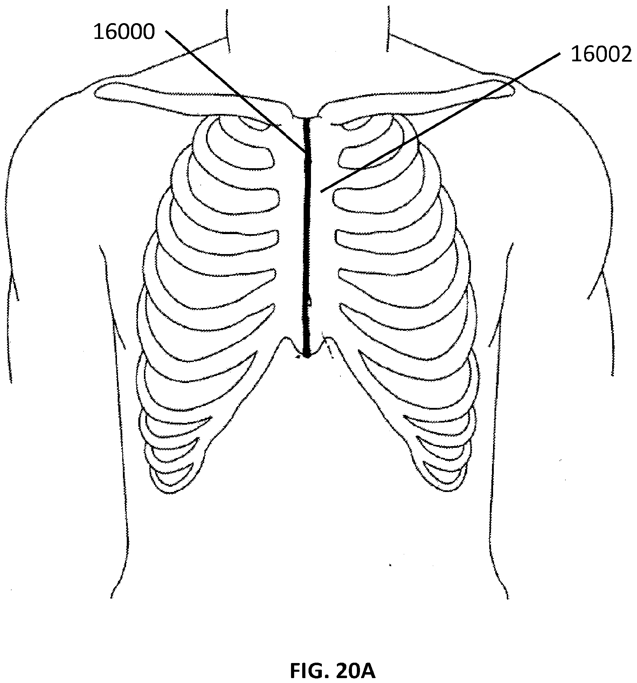

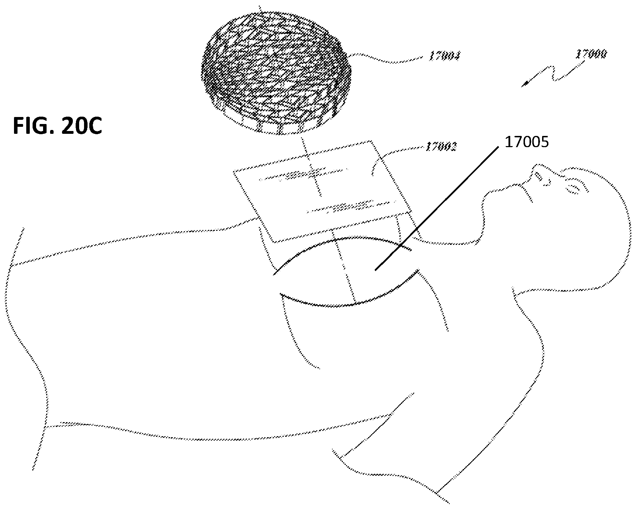

### The Method of Closing a Sternal Opening of Figures 20A-20C

A median sternotomy is a type of a surgical procedure in which a vertical incision is made along the sternum. Figure 20A depicts an embodiment of a sternal incision 16000, through the sternum of a human 16002. Such incisions may be completed with a sternal saw or other suitable cutting device. Such a sternal incision results in a separation of the two halves of the sternum, which may be expanded into a sternal opening allowing a physician to access the underlying organs, such as the heart. Sternal incisions are commonly used when performing open heart surgery. In certain embodiments, as described in greater detail below, a stabilizing structure such as those disclosed above in relation to Figures 2A-3E, 12-19, may be placed within the sternal opening to enhance closure of the sternum opening.

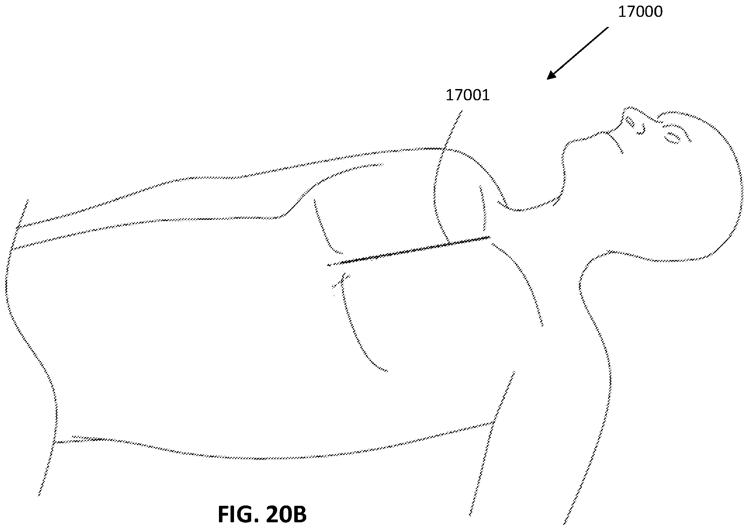

Figures 20B-C depicts a method 17000 of closing a sternal opening 17005 utilizing a stabilizing structure or wound closure device such as those disclosed above in relation to Figures 2A-3E, 12-19. As shown in Figure 20B, the sternal opening 17005 can be formed by a sternal incision 17001 through the sternum of a human such as described in relation with Figure 20A, and subsequent separation of the two halves of sternum. In some embodiments, an organ protection layer 17002, such as organ protection layers or tissue protection layers described herein this section or elsewhere in the specification, may be placed over the heart or any exposed tissue within the sternal opening 17005. In some embodiments, the organ protection layer 17002 can be provided with openings (not shown), such as holes, slits, or channels, to allow the removal of fluids from the opening 17005 or the transmittal of negative pressure to the opening 17005. In certain embodiments, the organ protection layer 17002 may be of a suitable stiffer material than typical organ protection layers, such as for example, a stiffened polymer. A stiffer organ protection layer may thereby better shield the underlying heart or other organs from the forces of NPWT. In embodiments, the organ protection layer may comprise hydrophilic PVA foam, or any suitable foam described herein this section or elsewhere in the specification.

A bottom layer of foam (not shown) may be optionally placed over the organ protection layer 17001. This bottom layer of foam may extend outward beneath the sternum, and optionally be attached to a stabilizing structure placed over the foam. In embodiments, the stabilizing structure may be positioned under the sternal opening, within the sternal opening or above the sternal opening. By extending outward from the stabilizing structure, the bottom layer of foam may prevent the stabilizing structure from being forced upward by pressure from the underlying organs.

In certain embodiments, the stabilizing structure 17004 is placed within the sternal opening, directly between the bones of the sternum. As with the stabilizing structures described elsewhere in the specification, such structures collapse under negative pressure, thereby serving to draw the edges of the sternal opening together. The stabilizing structure may be surrounded on the periphery by tissue anchors such that the tissue anchors engage periphery tissue of the opening 17005 and may facilitate closure of the opening 17005. As with the stabilizing structures disclosed above in relation to Figures 15A - 19, a stabilizing structure placed within the sternal opening may be re-sizeable to suitably fit the size and shape of the opening.

In some embodiments, an optional top layer of foam (not shown) may be applied to the top of the stabilizing structure. This top layer of foam may extend outward outside the sternal opening, and/or the top foam may be placed above the stabilizing structure but still within the sternal opening. In embodiments, a layer or layers of foam may be applied around the periphery of the stabilizing structure within the sternal opening.

In certain embodiments, a drape (not shown) may be applied to the top of the top foam, thereby forming an air-tight seal over the stabilizing structure, allowing for the application of negative pressure. Negative pressure may be applied to the stabilizing structure for any length of time described herein this section or elsewhere in the specification, for example about: 1 hour, 6 hours, 12 hours, 24 hours, 48 hours, or more than 48 hours.

In embodiments, stabilizing clips may be attached to the stabilizing structure, thereby serving to maintain the stabilizing structure in position between the bone of the sternum. In some embodiments, the stabilizing clips may clip directly to the sternum to maintain the stabilizing structure in place.

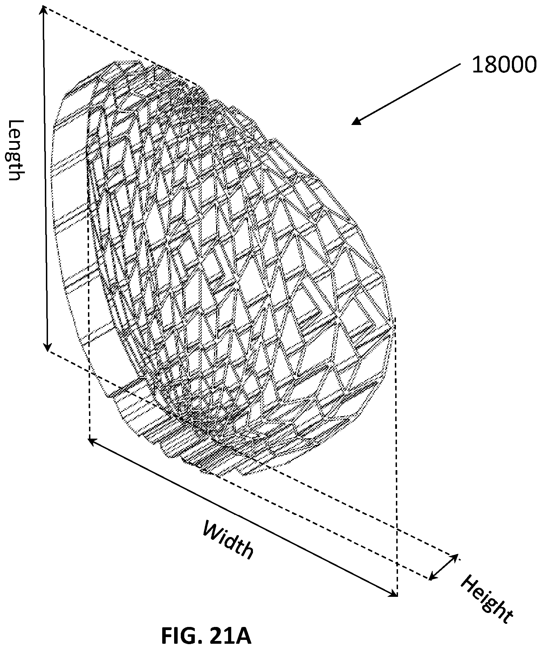

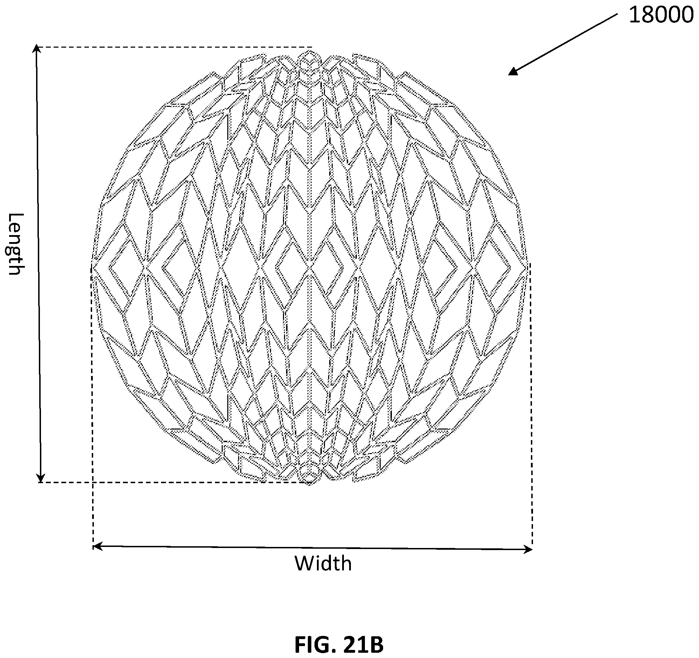

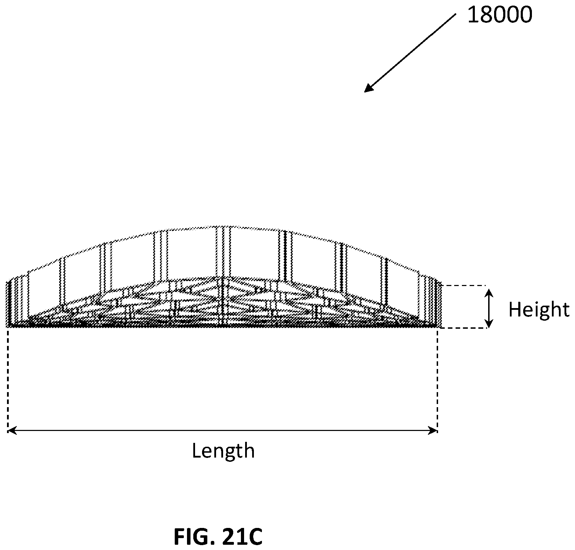

### The Wound Closure Systems and Apparatuses of Figures 21 A-22D

As discussed elsewhere in the specification, a stabilizing structure may be curved or bent along the horizontal plane, such that the stabilizing structure has a non-flat shape, such as a dome shape or a bowl shape.

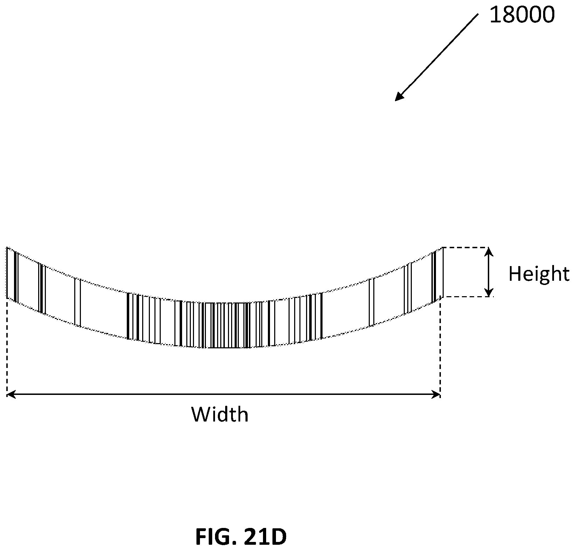

Figures 21A-D illustrate various views of an embodiment of a stabilizing structure 18000, similar to the stabilizing structures disclosed in relation with Figures 2A-2C, 9A, 12, 13A-14D. Figures 21A-D are a perspective view, a top view, a side view, and a front view of the stabilizing structure 18000, respectively, when the stabilizing structure 8000 is oriented such that length of the stabilizing structure is disposed from the front to the back, the width is disposed is disposed from the left side to the right side, and the height is disposed from the top to the bottom. Here, the stabilizing structure 18000 is curved along only its width. In some embodiments, the top surface and/or the bottom surface of the stabilizing structure 18000 may be curved along the width of the stabilizing structure such that they can better accommodate to the shape of a wound. Such curved stabilizing structure may be useful for the wound located in/on the curved surface. In certain embodiments, such as shown in Figures 21A-D, the stabilizing structure 18000 is shaped such that the upper surface is concave along its width while the bottom surface is convex along its width. The height may be constant across the stabilizing structure, as shown by Figure 21D. In certain embodiments, the upper surface of the stabilizing structure may convex along its width while the bottom surface may be concave along its width. In certain embodiments, both upper and bottom surfaces may be either concave or convex along its width. In some embodiments, either upper surface or bottom surface is curved along its width while the other surface is flat. Similarly, in some embodiments, the upper surface and/or the bottom surface may be curved along the length, instead of the width of the stabilizing structure.

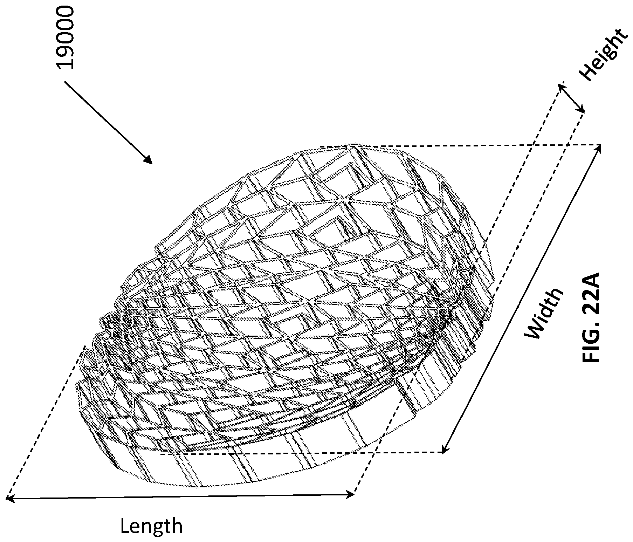

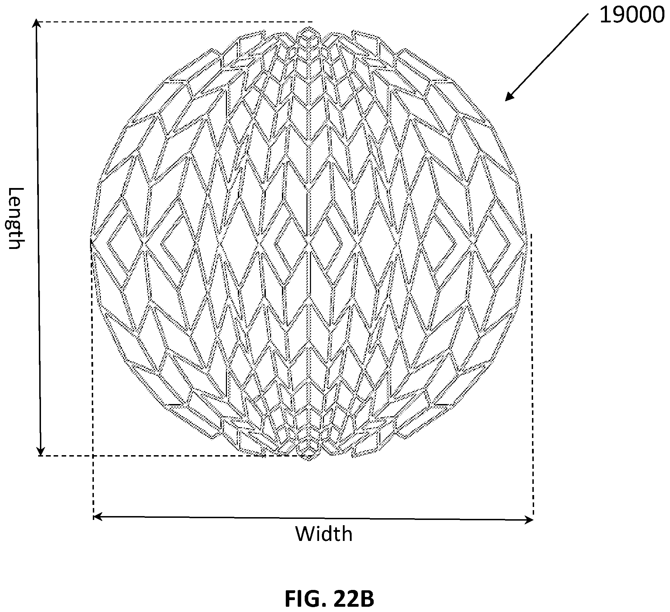

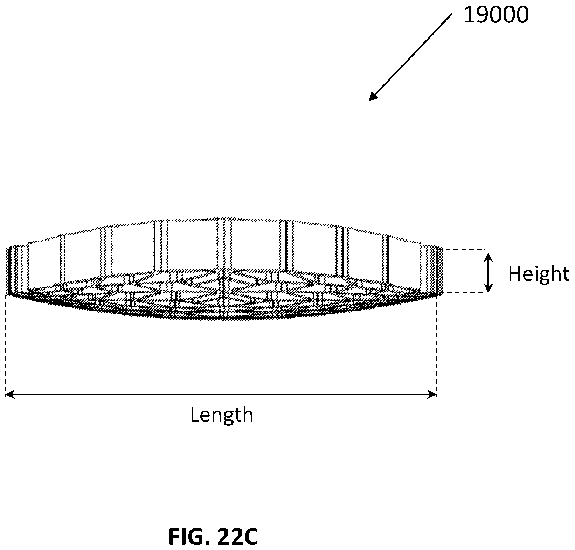

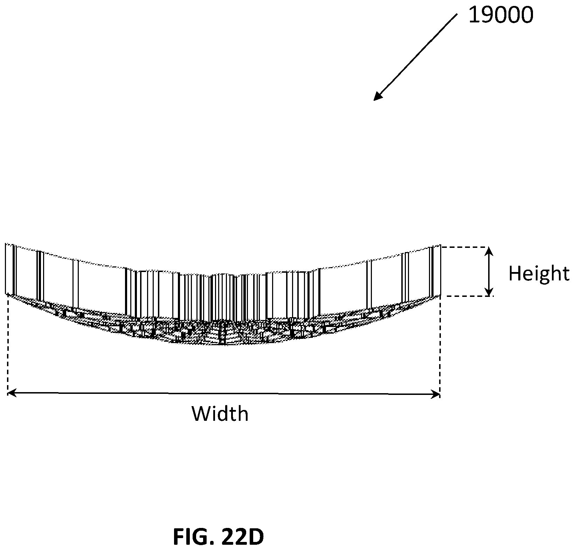

A stabilizing structure may be shaped such that the upper surface and/or the bottom surface of the stabilizing structure is curved both along the width and the length of the stabilizing structure. Figure 22A-B illustrates various views of such an embodiment of a stabilizing structure 19000 which is shaped such that the upper surface is concave and the bottom surface is convex along the width and the length of the stabilizing structure, thus the stabilizing structure is bowl-shaped. Figures 22A-D are a perspective view, a top view, a side view, and a front view of the stabilizing structure 19000, respectively, when the stabilizing structure 19000 is oriented such that length of the stabilizing structure is disposed from front to back, the width is disposed is disposed from left side to right side, and the height is disposed from top to bottom. In some embodiments, the upper surface is convex while the bottom surface is concave along the length and the width of the stabilizing structure. Both of upper and bottom surfaces may be either concave or convex along its length and width. In some embodiments, the upper surface and/or the lower surface may be convex along the length while concave along the width, or concave along the length while convex along the width. In some embodiments, only one of upper surface or bottom surface is curved along its width and its length while the other surface is flat.

Similar to wound closure devices described elsewhere in the specification, a wound closure device may include optional bottom, a top, a middle, and/or further layers of foam in addition to curved stabilizing structures described in relation with Figures 21A-22D, and any of these layers of foam may be curved along the length and or width of the stabilizing structure as well to better accommodate with the wound or the stabilizing structure. In some embodiments, the lower surface of the top layer of foam and/or the upper surface of the bottom layer of foam may be curved, such that layers of foam have tighter fit with the curved stabilizing structure. In some embodiments, layers of foam may be curved regardless of the stabilizing structure such that the wound closure device better accommodates with the wound site.

Any stabilizing structures and/or layers of foam described in this specification may be replaced with a curved stabilizing structure and/or layer of foam as described in this section or in relation with Figures 21A-22D. In some embodiments, stabilizing structures and/or layers of foam with different degrees of curvatures along horizontal plane may be packaged together in a kit, such that the practitioner may choose and/or assemble a wound closure device with appropriate shape to better accommodate the shape of the wound site.

### The Wound Closure Systems and Apparatuses with Variable Curvature

In addition to stabilizing structures curved along horizontal plane such as described elsewhere in the specification, in some instances, a stabilizing structure with variable curvature may be desired, since the shape and curvature of the wound may vary as a patient changes one's posture. For example, in the case of abdominal wound opening similar with the wound described in relation with Figure 7, a stabilizing structure may be needed to be bent along the length of the wound such that the stabilizing structure better accommodate to the wound when the patient changes posture, for example sits up.

In some embodiments, a stabilizing structure may bend along its length with or without application of force. For example, a stabilizing structure similar with those described in relation with Figures 2A-2C, 9A, 12, 13A-19, 21A-22D may have means to act as hinges which may enable the stabilizing structure to be bent and keep its bent shape along its length upon exert of force. For example, the stabilizing structure may have one or more V-shaped cuts along its elongate strips and/or intervening members, such that v-shaped cut(s) act as hinges to facilitate bending of the stabilizing structure along its length. In some embodiments, at least one of v-shaped cuts may be provided at or near nodes where elongate strips meet with intervening members. In some embodiments, at least on v-shaped cuts may be provided on elongate strips in the middle between nodes.

The size and number of v-shaped cuts along the elongate strips may vary depending on desired degree of bendability of the stabilizing structure. A stabilizing structure with larger and/or more v-shaped cuts may make the stabilizing structure more bendable, such that it may be suitable for patients with more activity or weaker strength. In some embodiments, v-shaped cuts are made in one direction, such that the stabilizing structure can be bent in one way. In some embodiments, cuts are made in both directions, such that the stabilizing structure can be bent in both ways. The stabilizing structure may have any other suitable means available to the skilled person in the field to make the stabilizing structure bendable, such as mechanical joints. In some embodiments, at least part of the stabilizing structure may be constructed from a material of sufficient flexibility, such that the stabilizing structure can be bent with or without means to act as hinges.

### The Wound Closure Systems and Apparatuses with Varying Thickness

As discussed elsewhere in the specification, a wound closure device may be placed in the wound, being aligned with the surrounding tissue. In some cases, the thickness of surrounding tissues of the wound, such as fat, skin or muscles may not be consistent along the wound, and accordingly, the depth of the wound may not be consistent as well. For example, an abdominal wound such as shown and described in relation with Figures 4, 6, 8A may have inconsistent depth as fat tissue is generally thicker toward pubis bone than near xiphoid. Further, for a patient with more body fat, the trend of increasing fat thickness toward pubis bone may be greater. On the other hand, in case of the abdominal wound of a patient having less body fat, fat tissue may be thicker in the middle while it may be thinner at both ends, toward pubis bone and xiphoid. For such wounds with inconsistent depth, a wound closure device having varying thickness along its length may better accommodate to the wound.

Like wound closure devices described elsewhere in the specification, the wound closure device with varying thickness may include a stabilizing structure and optional layer(s) of foam (e.g., bottom, middle and/or top layers of foam). In some embodiments, the stabilizing structure and/or any layers of foam similar with those described with regard to Figures 2A-2C, 9A-C, 12, 13A-19, 21A-22D may have varying thickness along the length and/or width of the wound closure device. For example, the stabilizing structure and/or any layers of foam may be thicker at one end and thinner at the other end of the length and/or the width of the wound closure device. In some embodiments, the stabilizing structure and/or any layers of foam may be thicker at both ends, while thinner in the middle. In some embodiments, the stabilizing structure and/or any layers of foam may be thinner at both ends, while thicker in the middle.

In some embodiments, stabilizing structures and/or layers of foam with different pattern of varying thickness may be packaged together in a kit, such that the practitioner may choose and/or assemble a wound closure device with appropriate stabilizing structures and/or layers of foam to better accommodate the shape of the wound site.

### Other Variations

Although this disclosure describes certain embodiments, it will be understood by those skilled in the art that many aspects of the methods and devices shown and described in the present disclosure may be differently combined and/or modified to form still further embodiments or acceptable examples. All such modifications and variations are intended to be included herein within the scope of this disclosure. Indeed, a wide variety of designs and approaches are possible and are within the scope of this disclosure. No feature, structure, or step disclosed herein is essential or indispensable. Moreover, while illustrative embodiments have been described herein, the scope of any and all embodiments having equivalent elements, modifications, omissions, combinations (e.g., of aspects across various embodiments), substitutions, adaptations and/or alterations as would be appreciated by those in the art based on the present disclosure. While certain embodiments have been described, these embodiments have been presented by way of example only, and are not intended to limit the scope of protection.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

The scope of the present disclosure is not intended to be limited by the specific disclosures of preferred embodiments in this section or elsewhere in this specification, and is defined by the appended claims.

The language of the claims is to be interpreted broadly based on the language employed in the claims and not limited to the examples described in the present specification or during the prosecution of the application, which examples are to be construed as non-exclusive.

## Claims

1. A wound closure device, comprising:
a stabilizing structure (2000) for insertion into a wound, the stabilizing structure configured to collapse more in a horizontal plane parallel to the length and width of the stabilizing structure than in a vertical plane perpendicular to the horizontal plane wherein the stabilizing structure comprises one or more cells (2004) and wherein the stabilizing structure is configured to collapse by collapsing the one or more cells;
a top layer of foam (3116) for positioning above the stabilizing structure, the top layer of foam conforming to a top surface of the stabilizing structure; and
a bottom layer of foam (3102) for positioning below the stabilizing structure, the bottom layer of foam conforming to a bottom surface of the stabilizing structure;
wherein the top layer of foam and/or the bottom layer of foam is at least partially curved along a horizontal plane parallel to a length and a width of the stabilizing structure.

2. The wound closure device of claim 1, wherein the stabilizing structure is at least partially curved along the horizontal plane.

3. The wound closure device of claim 1 or 2, wherein the top layer of the foam, the bottom layer of foam and/or the stabilizing structure is curved along the length but not the width of the stabilizing structure.

4. The wound closure device of any one of the preceding claims, wherein the top layer of the foam, the bottom layer of foam and/or the stabilizing structure is curved along the width but not the length of the stabilizing structure.

5. The wound closure device of any of the preceding claims, wherein the top layer of the foam, the bottom layer of foam and/or the stabilizing structure is curved along both the length and the width of the stabilizing structure.

6. The wound closure device of claim 5, wherein the top layer of the foam, the bottom layer of foam, and/or the stabilizing structure is dome-shaped.

7. The wound closure device of any of the preceding claims, wherein the stabilizing structure, the top layer of foam and the bottom layer of foam each has an oval or oculiform shape.

8. The wound closure device of claim 1, wherein the stabilizing structure is at least partially curved along a horizontal plane parallel to a length and width of the stabilizing structure.

9. The wound closure device of claim 8, wherein the stabilizing structure is curved only along the width but not the length of the stabilizing structure.

10. The wound closure device of claim 8, wherein the stabilizing structure is curved along both the width and the length of the stabilizing structure.

11. The wound closure device of any one of claims 8-10, wherein the stabilizing structure comprises one or more detachable segments.

12. The wound closure device of any one of claims 8-11, wherein the stabilizing structure has an oval or oculiform shape.

## Patentansprüche

1. Eine Wundverschlussvorrichtung, beinhaltend:
eine Stabilisierungsstruktur (2000) zum Einführen in eine Wunde, wobei die Stabilisierungsstruktur konfiguriert ist, um in einer horizontalen Ebene, die zu der Länge und Breite der Stabilisierungsstruktur parallel ist, stärker in sich zusammenzufallen als in einer vertikalen Ebene, die zu der horizontalen Ebene senkrecht ist, wobei die Stabilisierungsstruktur eine oder mehrere Zellen (2004) beinhaltet und wobei die Stabilisierungsstruktur konfiguriert ist, um durch das In-sich-Zusammenfallen der einen oder der mehreren Zellen in sich zusammenzufallen;
eine Oberschicht aus Schaumstoff (3116) zur Positionierung über der Stabilisierungsstruktur, wobei die Oberschicht aus Schaumstoff mit einer oberen Oberfläche der Stabilisierungsstruktur übereinstimmt; und
eine Unterschicht aus Schaumstoff (3102) zur Positionierung unter der Stabilisierungsstruktur, wobei die Unterschicht aus Schaumstoff mit einer unteren Oberfläche der Stabilisierungsstruktur übereinstimmt;
wobei die obere Schicht aus Schaumstoff und/oder die untere Schicht aus Schaumstoff entlang einer horizontalen Ebene, die zu einer Länge und einer Breite der Stabilisierungsstruktur parallel ist, mindestens teilweise gekrümmt ist.

2. Wundverschlussvorrichtung gemäß Anspruch 1, wobei die Stabilisierungsstruktur entlang der horizontalen Ebene mindestens teilweise gekrümmt ist.

3. Wundverschlussvorrichtung gemäß Anspruch 1 oder 2, wobei die Oberschicht aus dem Schaumstoff, die Unterschicht aus Schaumstoff und/oder die Stabilisierungsstruktur entlang der Länge, aber nicht der Breite der Stabilisierungsstruktur gekrümmt ist.

4. Wundverschlussvorrichtung gemäß einem der vorhergehenden Ansprüche, wobei die Oberschicht aus dem Schaumstoff, die Unterschicht aus Schaumstoff und/oder die Stabilisierungsstruktur entlang der Breite, aber nicht der Länge der Stabilisierungsstruktur gekrümmt ist.

5. Wundverschlussvorrichtung gemäß einem der vorhergehenden Ansprüche, wobei die Oberschicht aus dem Schaumstoff, die Unterschicht aus Schaumstoff und/oder die Stabilisierungsstruktur sowohl entlang der Länge als auch entlang der Breite der Stabilisierungsstruktur gekrümmt ist.

6. Wundverschlussvorrichtung gemäß Anspruch 5, wobei die Oberschicht aus dem Schaumstoff, die Unterschicht aus Schaumstoff und/oder die Stabilisierungsstruktur kuppelförmig ist.

7. Wundverschlussvorrichtung gemäß einem der vorhergehenden Ansprüche, wobei die Stabilisierungsstruktur, die Oberschicht aus Schaumstoff und die Unterschicht aus Schaumstoff jeweils eine ovale oder augenförmige Form aufweisen.

8. Wundverschlussvorrichtung gemäß Anspruch 1, wobei die Stabilisierungsstruktur entlang einer horizontalen Ebene, die zu einer Länge und Breite der Stabilisierungsstruktur parallel ist, mindestens teilweise gekrümmt ist.

9. Wundverschlussvorrichtung gemäß Anspruch 8, wobei die Stabilisierungsstruktur nur entlang der Breite, aber nicht der Länge der Stabilisierungsstruktur gekrümmt ist.

10. Wundverschlussvorrichtung gemäß Anspruch 8, wobei die Stabilisierungsstruktur sowohl entlang der Breite als auch entlang der Länge der Stabilisierungsstruktur gekrümmt ist.

11. Wundverschlussvorrichtung gemäß einem der Ansprüche 8-10, wobei die Stabilisierungsstruktur ein oder mehrere abnehmbare Segmente beinhaltet.

12. Wundverschlussvorrichtung gemäß einem der Ansprüche 8-11, wobei die Stabilisierungsstruktur eine ovale oder augenförmige Form aufweist.

## Revendications

1. Un dispositif de fermeture de plaie, comprenant :
une structure stabilisatrice (2000) destinée à être insérée dans une plaie, la structure stabilisatrice étant configurée pour s'écraser davantage dans un plan horizontal parallèle à la longueur et à la largeur de la structure stabilisatrice que dans un plan vertical perpendiculaire au plan horizontal, la structure stabilisatrice comprenant une ou plusieurs cellules (2004) et la structure stabilisatrice étant configurée pour s'écraser par écrasement de ces une ou plusieurs cellules ;
une couche supérieure de mousse (3116) destinée à être positionnée au-dessus de la structure stabilisatrice, la couche supérieure de mousse épousant la forme d'une surface supérieure de la structure stabilisatrice ; et
une couche inférieure de mousse (3102) destinée à être positionnée au-dessous de la structure stabilisatrice, la couche inférieure de mousse épousant la forme d'une surface inférieure de la structure stabilisatrice ;
dans lequel la couche supérieure de mousse et/ou la couche inférieure de mousse sont au moins partiellement incurvées le long d'un plan horizontal parallèle à une longueur et à une largeur de la structure stabilisatrice.

2. Le dispositif de fermeture de plaie de la revendication 1, dans lequel la structure stabilisatrice est au moins partiellement incurvée le long du plan horizontal.

3. Le dispositif de fermeture de plaie de la revendication 1 ou de la revendication 2, dans lequel la couche supérieure de la mousse, la couche inférieure de mousse et/ou la structure stabilisatrice sont incurvées le long de la longueur mais pas de la largeur de la structure stabilisatrice.

4. Le dispositif de fermeture de plaie de l'une quelconque des revendications précédentes, dans lequel la couche supérieure de la mousse, la couche inférieure de mousse et/ou la structure stabilisatrice sont incurvées le long de la largeur mais pas de la longueur de la structure stabilisatrice.

5. Le dispositif de fermeture de plaie de n'importe lesquelles des revendications précédentes, dans lequel la couche supérieure de la mousse, la couche inférieure de mousse et/ou la structure stabilisatrice sont incurvées le long aussi bien de la longueur que de la largeur de la structure stabilisatrice.

6. Le dispositif de fermeture de plaie de la revendication 5, dans lequel la couche supérieure de la mousse, la couche inférieure de mousse et/ou la structure stabilisatrice sont en forme de dôme.

7. Le dispositif de fermeture de plaie de n'importe lesquelles des revendications précédentes, dans lequel la structure stabilisatrice, la couche supérieure de mousse et la couche inférieure de mousse ont chacune une forme ovale ou oculiforme.

8. Le dispositif de fermeture de plaie de la revendication 1, dans lequel la structure stabilisatrice est au moins partiellement incurvée le long d'un plan horizontal parallèle à une longueur et à une largeur de la structure stabilisatrice.

9. Le dispositif de fermeture de plaie de la revendication 8, dans lequel la structure stabilisatrice est incurvée seulement le long de la largeur mais pas de la longueur de la structure stabilisatrice.

10. Le dispositif de fermeture de plaie de la revendication 8, dans lequel la structure stabilisatrice est incurvée le long aussi bien de la largeur que de la longueur de la structure stabilisatrice.

11. Le dispositif de fermeture de plaie de l'une quelconque des revendications 8 à 10, dans lequel la structure stabilisatrice comprend un ou plusieurs segments détachables.

12. Le dispositif de fermeture de plaie de l'une quelconque des revendications 8 à 11, dans lequel la structure stabilisatrice a une forme ovale ou oculiforme.
